(19) Europäisches Patentamt
European Patent Office
Office européen des brevets

(11) EP 1 177 791 A2

(12) EUROPEAN PATENT APPLICATION

(43) Date of publication:
06.02.2002 Bulletin 2002/06

(21) Application number: 01306440.7

(22) Date of filing: 27.07.2001

(51) Int Cl.7: **A61K 31/496**, A61K 31/404, A61K 31/422, A61K 31/5377, A61K 31/5355, A61K 31/00, A61K 45/06, A61P 35/00

(84) Designated Contracting States:
**AT BE CH CY DE DK ES FI FR GB GR IE IT LI LU MC NL PT SE TR**
Designated Extension States:
**AL LT LV MK RO SI**

(30) Priority: **31.07.2000 US 221717**

(71) Applicant: **Pfizer Products Inc.**
**Groton, CT 06340-5146 (US)**

(72) Inventor: **Krasner, Alan Seth**
**Groton, Connecticut 06340 (US)**

(74) Representative:
**Simpson, Alison Elizabeth Fraser et al**
**Urquhart-Dykes & Lord, 30 Welbeck Street**
**London W1G 8ER (GB)**

(54) **Use of glycogen phosphorylase inhibitors to inhibit tumor growth**

(57) This invention relates to the use of glycogen phosphorylase inhibitors of Formula I or Formula IA:

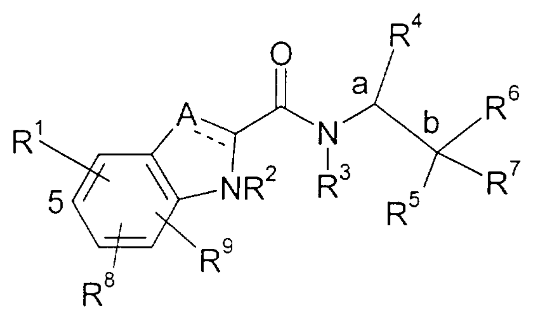

**Formula I**

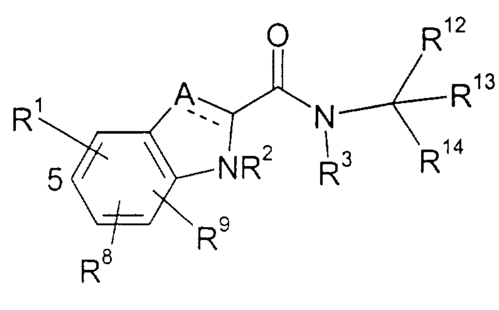

**Formula IA**

as defined herein, and their pharmaceutically acceptable salts and prodrugs thereof, to inhibit abnormal cell growth in mammals, including humans. The invention also relates to pharmaceutical compositions containing glycogen phosphorylase inhibitors alone or in combination with other glycogen phosphorylase inhibitors or other inhibitors of abnormal cell growth, and to methods of treating cancer, hyperproliferative disorders, or abnormal cell growth in a mammal by administering to a mammal in need thereof the compounds and compositions of the invention.

EP 1 177 791 A2

## Description

Field of the Invention

This invention relates to the use of glycogen phosphorylase inhibitors to inhibit tumor growth in mammals, including humans.

Background of the Invention

Glycogenolysis in tissues, whereby glycogen is cleaved to release glucose-1-phosphate, is catalyzed by glycogen phosphorylase (GP). In humans, three isoforms of this enzyme have been identified: the liver isoform (HLGP), the muscle isoform (HMGP), and the brain isoform (HBGP). These isoforms are products of three separate genes and have 80-83% amino acid identity (C. B Newgard, D. R. Littman, C. van Gendered, M. Smith, and R. J. Fletterick, J. Biol. Chem. 263:3850-3857, 1988). Glycogen phosphorylase is also present in bacteria.

Several authors have observed correlations between glycogen levels and various parameters of tumor growth. In 1980, for example, Rousset et al. reported their investigation of glycogen levels in several tumor cell lines in vitro and in vivo, finding that the highest glycogen content was associated with cell lines exhibiting the slowest tumor growth rate (J. Natl. Cancer Inst. 65(5):885-889, 1980). Numerous other investigators have also performed such studies, continuing to the present. For example, Yano et al. reported that the glycogen level in lung carcinomas was higher than in normal tissues, and that there was a correlation between glycogen levels degree of tumor differentiation (Yano, K. et al., Cancer Letters 110:29-34, 1996). Skwarski et al. reported that the glycogen content of gastric carcinomas was high relative to normal gastric mucosa, and suggested a link between the risk of gastric cancer and glycogen storage (Skwarski, L. et al., Cancer Letters 127:123-128, 1998). Even more recently, Takahashi et al, examined colorectal and normal tissues and observed that glycogen levels were higher in cancerous tissues, and lower in the normal tissues adjacent to the cancerous tissue, compared to outlying normal tissue (Takahashi et al. J. Gastroenterology 34 (4):474-480, 1999). These authors also observed that glycogen levels were highest in cancers with high proportions of cells in the $G_1$ phase of the cell cycle, and suggested that it may be possible that an artificial decrease in the amount of glycogen is capable of inhibiting cancer growth by keeping the cell cycle in the state of $G_1$ stop or $G_0$ (Id. at 479). Applicants believe that the progression from G1 to S, and the accompanying ability of the cancer cell to reproduce and cause tumor growth, requires glycogenolysis, i.e., the cleavage of glycogen to release glucose-1-phosphate. Accordingly, inhibition of tumor growth will be achieved by preventing glycogen breakdown by inhibiting glycogen phosphorylase, particularly in those cancers associated with increased glycogen levels, such as colorectal cancer and lung cancer.

Some examples of glycogen phosphorylase inhibitors that have been reported to date include glucose and glucose analogs (e.g., Martin, J. L. et al., Biochemistry 1991, 30, 10101), caffeine and other purine analogs (e.g., Kasvinsky, P. J. et al. J. Biol. Chem. 1978, 253, 3343-3351 and 9102-9106), and inhibitors of the type described by Oikonomakos, N. G. et al., Protein Sci. 1999, 8, 1930-1945. Compounds inhibiting glycogen phosphorylase are also reported in numerous patents and patent applications, e.g., WO 95/24391, WO 97/09040, WO 98/40353, WO 98/50359, WO 97/37901, EP-0884050, U.S. Patent No. 4,786,641.

Glycogen phosphorylase inhibitors are useful in the treatment of diabetes mellitus. For example, International Patent publications WO 96/39384 and WO 96/39385, both published December 12, 1996, describe use of substituted N-(indole-2-carbonyl-) amides and derivatives for treatment of diabetes. These compounds are also described as useful in treatment of atherosclerosis, hyperinsulinemia, hypercholesterolemia, hypertension, hyperlipidemia, and in prevention of myocardial ischemic injury.

U.S. Patent 5,952,322 describes the use of glycogen phosphorylase inhibitors, such as those described in WO 96/39384 and WO 96/39385, to reduce tissue damage associated with non-cardiac ischemia.

U.S. Patent 5,882,885, issued March 16, 1999 refers to antagonists and agonists of streptococcal glycogen phosphorylase as useful in the treatment of otitis media, conjunctivitis, pneumonia, bacteremia, meningitis, sinusitis, pleural empyema and endocarditis.

Summary of the Invention

The present invention relates to a method of inhibiting tumor growth in a mammal, including a human, comprising administering to the mammal an effective amount of a compound which is a glycogen phosphorylase inhibitor of Formula I or Formula IA. Compounds of the Formula I and Formula IA have the following structures:

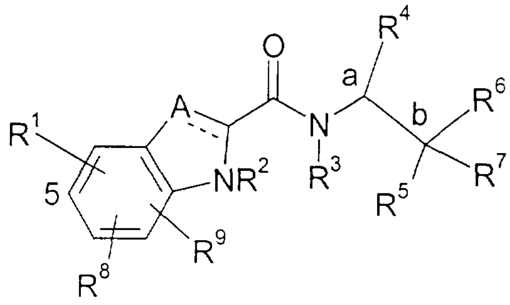

Formula I

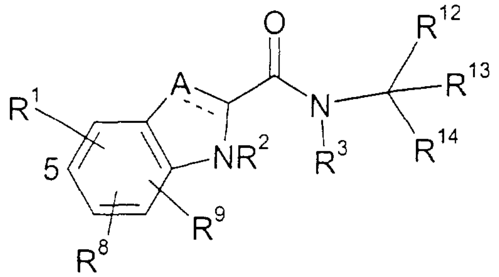

Formula IA

and the pharmaceutically acceptable salts and prodrugs thereof;
wherein:

the dotted line (---) is an optional bond;
A is —(CH)=, —C(($C_1$-$C_4$)alkyl)= or —C(halo)= when the dotted line (---) is a bond, or A is methylene or —CH(($C_1$-$C_4$)alkyl)- when the dotted line (---) is not a bond;
$R^1$, $R^8$ and $R^9$ are each independently H, halo, 4-, 6- or 7-nitro, cyano, ($C_1$-$C_4$)alkyl, ($C_1$-$C_4$)alkoxy, fluoromethyl, difluoromethyl or trifluoromethyl;
$R^2$ is H;
$R^3$ is H or ($C_1$-$C_5$)alkyl;
$R^4$ is H, methyl, ethyl, n-propyl, hydroxy($C_1$-$C_3$)alkyl, ($C_1$-$C_3$)alkoxy($C_1$-$C_3$)alkyl, phenyl($C_1$-$C_4$)alkyl, phenylhydroxy($C_1$-$C_4$)alkyl, phenyl($C_1$-$C_4$)alkoxy($C_1$-$C_4$)alkyl, thien-2- or-3-yl($C_1$-$C_4$)alkyl or fur-2- or -3-yl($C_1$-$C_4$)alkyl wherein said $R^4$ rings are mono-, di- or tri-substituted independently on carbon with H, halo, ($C_1$-$C_4$)alkyl, ($C_1$-$C_4$)alkoxy, trifluoromethyl, hydroxy, amino or cyano; or
$R^4$ is pyrid-2-, -3- or -4-yl($C_1$-$C_4$)alkyl, thiazol-2-, -4- or -5-yl($C_1$-$C_4$)alkyl, imidazol -1-,-2-, -4- or -5-yl($C_1$-$C_4$)alkyl, pyrrol-2- or -3-yl($C_1$-$C_4$)alkyl, oxazol-2-, -4- or -5-yl-($C_1$-$C_4$)alkyl, pyrazol-3-, -4- or -5-yl($C_1$-$C_4$)alkyl, isoxazol-3-, -4- or -5-yl($C_1$-$C_4$)alkyl, isothiazol-3-, -4- or -5-yl($C_1$-$C_4$)alkyl, pyridazin-3- or -4-yl-($C_1$-$C_4$)alkyl, pyrimidin-2-, -4-, -5- or -6-yl($C_1$-$C_4$)alkyl, pyrazin-2- or -3-yl($C_1$-$C_4$)alkyl or 1,3,5-triazin-2-yl($C_1$-$C_4$)alkyl, wherein said preceding $R^4$ heterocycles are optionally mono- or di-substituted independently with halo, trifluoromethyl, ($C_1$-$C_4$)alkyl, ($C_1$-$C_4$)alkoxy, amino or hydroxy and said mono-or di-substituents are bonded to carbon;
$R^5$ is H, hydroxy, fluoro, ($C_1$-$C_5$)alkyl, ($C_1$-$C_5$)alkoxy, ($C_1$-$C_6$)alkanoyl, amino($C_1$-$C_4$)alkoxy, mono-N- or di-N, N-($C_1$-$C_4$)alkylamino($C_1$-$C_4$)alkoxy, carboxy($C_1$-$C_4$)alkoxy, ($C_1$-$C_5$)alkoxy-carbonyl($C_1$-$C_4$)alkoxy, benzyloxycarbonyl($C_1$-$C_4$)alkoxy, or carbonyloxy wherein said carbonyloxy is carbon-carbon linked with phenyl, thiazolyl, imidazolyl, 1H-indolyl, furyl, pyrrolyl, oxazolyl, pyrazolyl, isoxazolyl, isothiazolyl, pyridazinyl, pyrimidinyl, pyrazinyl or 1,3,5-triazinyl and wherein said preceding $R^5$ rings are optionally mono-substituted with halo, ($C_1$-$C_4$)alkyl, ($C_1$-$C_4$)alkoxy, hydroxy, amino or trifluoromethyl and said mono-substituents are bonded to carbon;
$R^7$ is H, fluoro or ($C_1$-$C_5$)alkyl; or
$R^5$ and $R^7$ can be taken together to be oxo;
$R^6$ is C(O)$R^{10}$, C(O)N$R^{16}R^{17}$, ($C_1$-$C_8$)alkoxycarbonyl or carboxy;

wherein $R^{16}$ is H, hydroxy, or ($C_1$-$C_3$ alkoxy), wherein $R^{17}$ is H, ($C_1$-$C_8$)alkyl, hydroxy, ($C_1$-$C_8$)alkoxy, methylene-perfluorinated($C_1$-$C_8$) alkyl, phenyl, pyridyl, thienyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, pyridazinyl, pyrimidinyl, pyrazinyl, piperazinyl or 1,3,5-triazinyl wherein said preceding $R^{17}$ rings are carbon-nitrogen linked; or $R^{17}$ is mono-, di-or tri-substituted ($C_1$-$C_5$)alkyl, wherein said substituents are independently H, hydroxy, amino, mono-N- or di-N, N-($C_1$-$C_5$)alkylamino; or $R^{17}$ is mono- or di-substituted ($C_1$-$C_5$ alkyl), wherein said substituents are independently phenyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazolinyl, piperazinyl or 1,3,5-triazolinyl, wherein the nonaromatic nitrogen-containing $R^{17}$ rings are optionally mono-substituted on nitrogen with ($C_1$-$C_6$)alkyl, benzyl, benzoyl or ($C_1$-$C_6$)alkoxycarbonyl and wherein the $R^{17}$ rings are optionally mono-substituted on carbon with halo, ($C_1$-$C_4$)alkyl, ($C_1$-$C_4$)alkoxy, hydroxy, amino, or mono-N- or di-N,N($C_1$-$C_5$)alkylamino provided that no quaternized nitrogen is included and there are no nitrogen-oxygen, nitrogen-nitrogen or nitrogen-halo bonds;

$R^{10}$ is piperazin-1-yl, 4-($C_1$-$C_4$)alkylpiperazin-1-yl, 4-formylpiperazin-1-yl, morpholino, thiomorpholino, 1-oxothiomorpholino, 1,1-dioxo-thiomorpholino, thiazolidin-3-yl, 1-oxo-thiazolidin-3-yl, 1,1-dioxo-thiazolidin-3-yl, 2-($C_1$-$C_6$) alkoxycarbonylpyrrolidin-1-yl, oxazolidin-3-yl or 2(R)-hydroxymethylpyrrolidin-1-yl; or

$R^{10}$ is 3- and/or 4-mono-or di-substituted oxazetidin-2-yl, 2-, 4-, and/or 5- mono- or di-substituted oxazolidin-3-yl, 2-, 4-, and/or 5- mono- or di- substituted thiazolidin-3-yl, 2-, 4-, and/or 5- mono- or di- substituted 1-oxothiazolidin-3-yl, 2-, 4-, and/or 5- mono- or di-substituted 1,1-dioxothiazolidin-3-yl, 3- and/or 4-, mono- or di-substituted pyrrolidin-1-yl, 3-, 4-and/or 5-, mono-, di- or tri-substituted piperidin-1-yl, 3-, 4-, and/or 5- mono-, di-, or tri-substituted piperazin-1-yl, 3-substituted azetidin-1-yl, 4- and/or 5-, mono- or di-substituted 1,2-oxazinan-2-yl, 3-and/or 4-mono- or di-substituted pyrazolidin-1-yl, 4- and/or 5-, mono- or di-substituted isoxazolidin-2-yl, 4- and/or 5-, mono- and/or di-substituted isothiazolidin-2-yl wherein said $R^{10}$ substituents are independently H, halo, ($C_1$-$C_5$)-alkyl, hydroxy, amino, mono-N- or di-N,N-($C_1$-$C_5$)alkylamino, formyl, oxo, hydroxyimino, ($C_1$-$C_5$)alkoxy, carboxy, carbamoyl, mono-N-or di-N,N-($C_1$-$C_4$)alkylcarbamoyl, ($C_1$-$C_4$)alkoxyimino, ($C_1$-$C_4$)alkoxymethoxy, ($C_1$-$C_6$)alkoxycarbonyl, carboxy($C_1$-$C_5$)alkyl or hydroxy($C_1$-$C_5$)alkyl;

$R^{12}$ is H, methyl, ethyl, n-propyl, hydroxy($C_1$-$C_3$)alkyl, ($C_1$-$C_3$)alkoxy($C_1$-$C_3$)alkyl, phenyl($C_1$-$C_4$)alkyl, phenylhydroxy($C_1$-$C_4$)alkyl, (phenyl)(($C_1$-$C_4$)-alkoxy)($C_1$-$C_4$)alkyl, thien-2-or -3-yl($C_1$-$C_4$)alkyl or fur-2- or -3-yl($C_1$-$C_4$)alkyl wherein said $R^{12}$ rings are mono-, di- or tri-substituted independently on carbon with H, halo, ($C_1$-$C_4$)alkyl, ($C_1$-$C_4$) alkoxy, trifluoromethyl, hydroxy, amino, cyano or 4,5-dihydro-1H-imidazol-2-yl; or

$R^{12}$ is pyrid-2-, -3- or -4-yl($C_1$-$C_4$)alkyl, thiazol-2-, -4- or -5-yl($C_1$-$C_4$)alkyl, imidazol-2-,-4- or -5-yl($C_1$-$C_4$)alkyl, pyrrol-2- or -3-yl($C_1$-$C_4$)alkyl, oxazol-2-, -4- or -5-yl($C_1$-$C_4$)alkyl, pyrazol-3-, -4- or -5-yl($C_1$-$C_4$)alkyl, isoxazol-3-, -4- or -5-yl($C_1$-$C_4$)alkyl, isothiazol-3-, -4- or -5-yl($C_1$-$C_4$)alkyl, pyridazin-3- or -4-yl($C_1$-$C_4$)alkyl, pyrimidin-2-, -4-, -5- or -6-yl($C_1$-$C_4$)alkyl, pyrazin-2-or -3-yl($C_1$-$C_4$)alkyl, 1,3,5-triazin-2-yl($C_1$-$C_4$)alkyl or indol-2-($C_1$-$C_4$)alkyl, wherein said preceding $R^{12}$ heterocycles are optionally mono- or di-substituted independently with halo, trifluoromethyl, ($C_1$-$C_4$) alkyl, ($C_1$-$C_4$)alkoxy, amino, hydroxy or cyano and said substituents are bonded to carbon, or

$R^{12}$ is $R^{11}$-carbonyloxymethyl, wherein said $R^{11}$ is phenyl, thiazolyl, imidazolyl, 1H-indolyl, furyl, pyrrolyl, oxazolyl, pyrazolyl, isoxazolyl, isothiazolyl, pyridyl, pyridazinyl, pyrimidinyl, pyrazinyl or 1,3,5-triazinyl and wherein said preceding $R^{11}$ rings are optionally mono- or di-substituted independently with halo, amino, hydroxy, ($C_1$-$C_4$)alkyl, ($C_1$-$C_4$)alkoxy or trifluoromethyl and said mono- or di-substituents are bonded to carbon;

$R^{13}$ is H, methyl, ethyl, n-propyl, hydroxymethyl, or hydroxyethyl;

$R^{14}$ is C(O)$R^{15}$, carboxy, ($C_1$-$C_8$)alkoxycarbonyl, benzyloxycarbonyl, or CON$R^{18}R^{19}$;

$R^{15}$ is morpholino, thiomorpholino, 1-oxothiomorpholino, 1,1-dioxothiomorpholino, thiazolidin-3-yl, 1-oxothiazolidin-3-yl, 1,1-dioxothiazolidin-3-yl, pyrrolidin-1-yl, piperidin-1-yl, piperazin-1-yl, piperazin-4-yl, azetidin-1-yl, 1,2-oxazinan-2-yl, pyrazolidin-1-yl, isoxazolidin-2-yl, isothiazolidin-2-yl, 1,2-oxazetidin-2-yl, oxazolidin-3-yl, 3,4-dihydroisoquinolin-2-yl, 1,3-dihydroisoindol-2-yl, 3,4-dihydro-2H-quinol-1-yl, 2,3-dihydro-benzo[1,4]oxazin-4-yl, 2,3-dihydro-benzo[1,4]-thiazine-4-yl, 3,4-dihydro-2H-quinoxalin-1-yl, 3,4-dihydro-benzo[c][1,2]oxazin-1-yl, 1,4-dihydrobenzo[d][1,2]oxazin-3-yl, 3,4-dihydro-benzo[e][1,2]-oxazin-2-yl, 3H-benzo[d]isoxazol-2-yl, 3H-benzo[c]isoxazol-1-yl or azepan-1-yl,

wherein said $R^{15}$ rings are optionally mono-, di- or tri-substituted independently with halo, ($C_1$-$C_5$)alkyl, ($C_1$-$C_5$) alkoxy, hydroxy, amino, mono-N- or di-N,N-($C_1$-$C_5$)alkylamino, formyl, carboxy, carbamoyl, mono-N- or di-N, N-($C_1$-$C_5$)alkylcarbamoyl, ($C_1$-$C_6$)alkoxy($C_1$-$C_3$)alkoxy, ($C_1$-$C_5$)alkoxycarbonyl, benzyloxycarbonyl, ($C_1$-$C_5$)alkoxycarbonyl($C_1$-$C_5$)alkyl, ($C_1$-$C_4$)alkoxycarbonylamino, carboxy($C_1$-$C_5$)alkyl, carbamoyl($C_1$-$C_5$)alkyl, mono-N- or di-N,N-($C_1$-$C_5$)alkylcarbamoyl($C_1$-$C_5$)alkyl, hydroxy($C_1$-$C_5$)alkyl, ($C_1$-$C_4$)alkoxy($C_1$-$C_4$)alkyl, amino($C_1$-$C_4$)alkyl, mono-N- or di-N,N-($C_1$-$C_4$)alkylamino($C_1$-$C_4$)alkyl, oxo, hydroxyimino or ($C_1$-$C_6$)alkoxyimino and wherein no more than two substituents are selected from oxo, hydroxyimino or ($C_1$-$C_6$)alkoxyimino and oxo, hydroxyimino or ($C_1$-$C_6$) alkoxyimino are on nonaromatic carbon, and

wherein said $R^{15}$ rings are optionally additionally mono- or di-substituted independently with $(C_1-C_5)$alkyl or halo; wherein $R^{18}$ is H, $(C_1-C_6)$alkyl, cyclo$(C_3-C_6)$alkyl, cyclo$(C_3-C_6)$alkyl$(C_1-C_5)$alkyl, hydroxy or $(C_1-C_8)$alkoxy; and wherein $R^{19}$ is H, cyclo$(C_3-C_8)$alkyl, cyclo$(C_3-C_8)$alkyl$(C_1-C_5)$alkyl, cyclo$(C_4-C_7)$alkenyl, cyclo$(C_3-C_7)$alkyl$(C_1-C_5)$alkoxy, cyclo$(C_3-C_7)$alkoxy, hydroxy, methylene-perfluorinated$(C_1-C_8)$alkyl, phenyl, or a heterocycle wherein said heterocycle is pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl, 1,3,5-triazinyl, benzothiazolyl, benzoxazolyl, benzimidazolyl, thiochromanyl or tetrahydrobenzothiazolyl wherein said heterocycle rings are carbon-nitrogen linked, or $R^{19}$ is $(C_1-C_6)$alkyl or $(C_1-C_8)$alkoxy wherein said $(C_1-C_8)$alkoxy is optionally monosubstituted with cyclo$(C_4-C_7)$alken-1-yl, phenyl, thienyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, thiomorpholinyl, 1-oxo-thiomorpholinyl, 1,1-dioxothiomorpholinyl, pyridazinyl, pyrimidinyl, pyrazolinyl, piperazinyl, 1,3,5-triazinyl or indolyl and wherein said $(C_1-C_6)$alkyl or $(C_1-C_8)$alkoxy are optionally additionally independently mono- or di-substituted with halo, hydroxy, $(C_1-C_5)$alkoxy, amino, mono-N- or Di-N,N-$(C_1-C_5)$alkylamino, cyano, carboxy, or $(C_1-C_4)$alkoxycarbonyl, and wherein the $R^{19}$ rings are optionally mono- or di-substituted independently on carbon with halo, $(C_1-C_4)$alkyl, $(C_1-C_4)$alkoxy, hydroxy, hydroxy$(C_1-C_4)$alkyl, amino$(C_1-C_4)$alkyl, mono-N- or di-N,N-$(C_1-C_4)$alkylamino, cyano, carboxy, $(C_1-C_5)$alkoxycarbonyl, carbamoyl, formyl or trifluoromethyl and said $R^{19}$ rings may optionally be additionally mono- or di-substituted independently with $(C_1-C_5)$alkyl or halo, with the proviso that no quaternized nitrogen on any $R^{19}$ heterocycle is included, with the further provisos that when $R^{12}$ is benzyl and $R^{13}$ is methyl, $R^{15}$ is not 4-hydroxy-piperidin-1-yl, and when $R^{12}$ is benzyl and $R^{13}$ is methyl, $R^{15}$ is not $C(O)N(CH_3)_2$.

The invention also relates to the use of a compound of formula (I) or (IA), including all the embodiments, preferred embodiments, more preferred embodiments, especially preferred embodiments and particularly preferred embodiments thereof, in the manufacture of a medicament for the inhibition of tumor growth in a mammal, including a human.

In an embodiment, the method comprises administering to a mammal, including a human, an effective amount of a first group of preferred compounds of Formula I consisting of those compounds wherein:

$R^1$ is 5-H, 5-halo, 5-methyl, 5-cyano or 5-trifluoromethyl;
$R^8$ and $R^9$ are each independently H or halo;
A is —C(H)=;
$R^2$ and $R^3$ are H;
$R^4$ is phenyl$(C_1-C_2)$alkyl wherein said phenyl groups are mono-, di- or tri-substituted independently with H or halo or mono- or di- substituted independently with H, halo, $(C_1-C_4)$alkyl, $(C_1-C_4)$alkoxy, trifluoromethyl, hydroxy, amino or cyano, or
$R^4$ is thien-2- or -3-yl$(C_1-C_2)$alkyl, pyrid-2-, -3- or -4-yl$(C_1-C_2)$alkyl, thiazol-2-, -4- or -5-yl$(C_1-C_2)$alkyl, imidazol -1-, -2-, -4- or -5-yl$(C_1-C_2)$alkyl, fur-2- or -3-yl$(C_1-C_2)$alkyl, pyrrol-2- or-3-yl$(C_1-C_2)$alkyl, oxazol-2-, -4- or -5-yl-$(C_1-C_2)$alkyl, pyrazol-3-, -4- or -5-yl$(C_1-C_2)$alkyl, isoxazol-3-, -4- or -5-yl$(C_1-C_2)$alkyl, wherein said preceding $R^4$ heterocycles are optionally mono- or di-substituted independently with halo, trifluoromethyl, $(C_1-C_4)$alkyl, $(C_1-C_4)$alkoxy, amino or hydroxy and said mono- or di-substituents are bonded to carbon;
$R^5$ is hydroxy;
$R^6$ is $C(O)R^{10}$; and
$R^7$ is H.

In another embodiment, the method comprises administering to a mammal, including a human, an effective amount of an especially preferred group of compounds within the first group of preferred compounds, consisting of those compounds of Formula I wherein:

the carbon atom labelled (a) has (S) stereochemistry;
the carbon atom labelled (b) has (R) stereochemistry;
$R^4$ is phenyl$(C_1-C_2)$alkyl, thien-2-yl-$(C_1-C_2)$alkyl, thien-3-yl-$(C_1-C_2)$alkyl, fur-2-yl-$(C_1-C_2)$alkyl or fur-3-yl-$(C_1-C_2)$alkyl, wherein said rings are mono- or di- substituted independently with H or fluoro; and
$R^{10}$ is morpholino, 4-$(C_1-C_4)$alkylpiperazin-1-yl, 3-substituted azetidin-1-yl, 3- and/or 4-, mono- or di-substituted pyrrolidin-1-yl, 4- and/or 5- mono- or di-substituted isoxazolidin-2-yl, 4- and/or 5-, mono- or di-substituted 1,2-oxazinan-2-yl wherein said substituents are each independently H, halo, hydroxy, amino, mono-N- or di-N,N-$(C_1-C_6)$alkylamino, oxo, hydroxyimino or alkoxy.

In a still further embodiment, the method comprises administering to a mammal, including a human, an effective amount of a particularly preferred group of compounds within the above group of especially preferred compounds, consisting of the compounds:

5-Chloro-1H-indole-2-carboxylic acid [(1S)-benzyl-(2R)-hydroxy-3-(4-methyl-piperazin-1-yl)-3-oxo-propyl]-amide hydrochloride,
5-Chloro-1H-indole-2-carboxylic acid [(1S)-benzyl-(2R)-hydroxy-3-(3-hydroxy-azetidin-1-yl)-3-oxo-propyl]-amide,
5-Chloro-1H-indole-2-carboxylic acid ((1S)-benzyl-(2R)-hydroxy-3-isoxazolidin-2-yl-3-oxo-propyl)-amide,
5-Chloro-1H-indole-2-carboxylic acid ((1S)-benzyl-(2R)-hydroxy-3-[1,2]oxazinan-2-yl-3-oxo-propyl)-amide,
5-Chloro-1H-indole-2-carboxylic acid [(1S)-benzyl-(2R)-hydroxy-3-((3S)-hydroxy-pyrrolidin-1-yl)-3-oxo-propyl]-amide,
5-Chloro-1H-indole-2-carboxylic acid [(1S)-benzyl-3-((3S,4S)-dihydroxy-pyrrolidin-1-yl)-(2R)-hydroxy-3-oxo-propyl]-amide,
5-Chloro-1H-indole-2-carboxylic acid [(1S)-benzyl-3-(cis-3,4-dihydroxy-pyrrolidin-1-yl)-(2R)-hydroxy-3-oxo-propyl]-amide; and
5-Chloro-1H-indole-2-carboxylic acid ((1S)-benzyl-(2R)-hydroxy-3-morpholin-4-yl-3-oxo-propyl)-amide.

In yet another embodiment, the method comprises administering to a mammal, including a human, an effective amount of another particularly preferred group of compounds within the above group of especially preferred compounds, wherein $R^1$ is 5-chloro, $R^8$ and $R^9$ are both H, $R^4$ is benzyl; and $R^{10}$ is selected from the group consisting of4-methylpiperazin-1-yl, 3-hydroxyazetidin-1-yl, isoxazolidin-2-yl, (1,2)-oxazinan-2-yl, 3(S)-hydroxypyrrolidin-1-yl, (3S,4S)-dihydroxypyrrolidin-1-yl, cis-3,4-dihydroxypyrrolidin-1-yl; and morpholino.

In a still further embodiment, the method comprises administering to a mammal, including a human, an effective amount of a particularly preferred group of compounds within the above group of especially preferred compounds, consisting of the compounds:

5-Chloro-1H-indole-2-carboxylic acid [(1S)-benzyl-(2R)-hydroxy-3-((3S)-hydroxy-pyrrolidin-1-yl)-3-oxo-propyl]-amide,
5-Chloro-1H-indole-2-carboxylic acid [(1S)-benzyl-3-((3S,4S)-dihydroxy-pyrrolidin-1-yl)-(2R)-hydroxy-3-oxo-propyl]-amide, and
5-Chloro-1H-indole-2-carboxylic acid [(1S)-benzyl-3-(cis-3,4-dihydroxy-pyrrolidin-1-yl)-(2R)-hydroxy-3-oxo-propyl]-amide.

In an embodiment, the method comprises administering to a mammal, including a human, an effective amount of a second group of preferred compounds, consisting of those compounds of Formula I, wherein:

$R^1$ is H, halo, methyl or cyano;
$R^8$ and $R^9$ are each independently H or halo;
A is —C(H)=;
$R^2$ and d $R^3$ are H;
$R^4$ is phenyl($C_1$-$C_2$)alkyl wherein said phenyl groups are mono-, di- or tri-substituted independently with H or halo or mono- or di- substituted independently with H, halo, ($C_1$-$C_4$)alkyl, ($C_1$-$C_4$)alkoxy, trifluoromethyl, hydroxy, amino or cyano; or
$R^4$ is thien-2- or -3-yl($C_1$-$C_2$)alkyl, pyrid-2-, -3- or -4-yl($C_1$-$C_2$)alkyl, thiazol-2-, -4- or -5-yl($C_1$-$C_2$)alkyl, imidazol -1-, -2-, -4- or -5-yl($C_1$-$C_2$)alkyl, fur-2- or -3-yl($C_1$-$C_2$)alkyl, pyrrol-2- or-3-yl($C_1$-$C_2$)alkyl, oxazol-2-, -4- or -5-yl-($C_1$-$C_2$)alkyl, pyrazol-3-, -4- or -5-yl($C_1$-$C_2$)alkyl, isoxazol-3-, -4- or -5-yl($C_1$-$C_2$)alkyl wherein said preceding $R^4$ heterocycles are optionally mono- or di-substituted independently with halo, trifluoromethyl, ($C_1$-$C_4$)alkyl, ($C_1$-$C_4$)alkoxy, amino or hydroxy and said mono- or di-substituents are bonded to carbon;
$R^5$ is fluoro, ($C_1$-$C_4$)alkyl, ($C_1$-$C_5$)alkoxy, amino($C_1$-$C_4$)alkoxy, mono-N- or di-N,N-($C_1$-$C_4$)alkylamino($C_1$-$C_4$)alkoxy, carboxy($C_1$-$C_4$)alkoxy, ($C_1$-$C_5$)alkoxy-carbonyl($C_1$-$C_4$)alkoxy, benzyloxycarbonyl($C_1$-$C_4$)alkoxy;
$R^6$ is C(O)$R^{10}$; and
$R^7$ is H, fluoro or ($C_1$-$C_6$)alkyl.

In an embodiment, the method comprises administering to a mammal, including a human, an effective amount of a first group of preferred compounds of Formula IA, consisting of those compounds of Formula IA wherein:

$R^1$ is 5-H, 5-halo, 5-methyl, 5-cyano or 5-trifluoromethyl;
$R^8$ and $R^9$ are each independently H or halo;
A is —C(H)=;

$R^2$ and $R^3$ are H;

$R^{12}$ is H, methyl, phenyl($C_1$-$C_2$)alkyl, wherein said phenyl groups are mono- or di-substituted independently with H, halo, ($C_1$-$C_4$)alkyl, ($C_1$-$C_4$)alkoxy, trifluoromethyl, hydroxy, amino or cyano and wherein said $R^{12}$ groups are optionally additionally mono-substituted with halo; or

$R^{12}$ is thien-2- or -3-yl($C_1$-$C_2$)alkyl, pyrid-2-, -3- or -4-yl($C_1$-$C_2$)alkyl, thiazol-2-, -4- or-5-yl($C_1$-$C_2$)alkyl, imidazol-2-, -4- or -5-yl($C_1$-$C_2$)alkyl, fur-2- or -3-yl($C_1$-$C_2$)alkyl, pyrrol-2- or -3-yl($C_1$-$C_2$)alkyl, oxazol-2-, -4- or -5-yl($C_1$-$C_2$)alkyl, pyrazol-3-, -4- or -5-yl($C_1$-$C_2$)alkyl, isoxazol-3-, -4- or -5-yl($C_1$-$C_2$)alkyl, isothiazol-3-, -4- or -5-yl($C_1$-$C_2$)alkyl, pyridazin-3- or -4-yl($C_1$-$C_2$)alkyl, pyrimidin-2-, -4-, -5- or -6-yl($C_1$-$C_2$)alkyl, pyrazin-2- or -3-yl($C_1$-$C_2$)alkyl or 1,3,5-triazin-2-yl($C_1$-$C_2$)alkyl, wherein said preceding $R^{12}$ heterocycles are optionally mono- or di-substituted independently with halo, trifluoromethyl, ($C_1$-$C_4$)alkyl, ($C_1$-$C_4$)alkoxy, amino or hydroxy and said mono- or di-substituents are bonded to carbon;

$R^{13}$ is H; and

$R^{14}$ is C(O)$R^{15}$.

In another embodiment, the method comprises administering to a mammal, including a human, an effective amount of an especially preferred group of compounds within the first group of preferred compounds of Formula IA, consisting of those compounds:

$R^{12}$ is H, phenyl($C_1$-$C_2$)alkyl, thien-2- or -3-yl($C_1$-$C_2$)alkyl, fur-2- or -3-yl($C_1$-$C_2$)alkyl wherein said $R^{12}$ rings are mono- or di-substituted independently with H or fluoro; and

$R^{15}$ is morpholino, thiomorpholino, 1-oxothiomorpholino, 1,1-dioxothiomorpholino, thiazolidin-3-yl, 1 -oxothiazolidin-3-yl, 1,1-dioxothiazolidin-3-yl, pyrrolidin-1-yl, piperidin-1-yl, piperazin-1-yl, piperazin-4-yl, azetidin-1-yl, 1,2-oxazinan-2-yl, isoxazolidin-2-yl, isothiazolidin-2-yl, 1,2-oxazetidin-2-yl, oxazolidin-3-yl, 1,3-dihydroisoindol-2-yl, or azepan-1-yl,

wherein said $R^{15}$ rings are optionally mono- or di-substituted independently with halo, ($C_1$-$C_5$)alkyl, ($C_1$-$C_5$)alkoxy, hydroxy, amino, mono-N-or di-N,N-($C_1$-$C_5$)alkylamino, formyl, carboxy, carbamoyl, mono-N- or di-N,N-($C_1$-$C_5$) alkylcarbamoyl, ($C_1$-$C_5$)alkoxycarbonyl, hydroxy($C_1$-$C_5$)alkyl, amino($C_1$-$C_4$)alkyl, mono-N- or di-N,N-($C_1$-$C_4$) alkylamino($C_1$-$C_4$)alkyl, oxo, hydroxyimino or ($C_1$-$C_6$)alkoxyimino with the proviso that only the $R^{15}$ heterocycles thiazolidin-3-yl, pyrrolidin-1-yl, piperidin-1-yl, piperazin-1-yl, piperazin-4-yl, azetidin-1-yl, 1,2-oxazinan-2-yl, isoxazolidin-2-yl, or oxazolidin-3-yl are optionally mono- or di-substituted with oxo, hydroxyimino, or ($C_1$-$C_6$)alkoxyimino, and

wherein said $R^{15}$ rings are optionally additionally mono- or di-substituted independently with ($C_1$-$C_5$)alkyl.

In a further embodiment, the method comprises administering to a mammal, including a human, an effective amount of the particularly preferred compounds within the especially preferred compounds of Formula IA, consisting of the compounds:

5-Chloro-1H-indole-2-carboxylic acid [(1S)-benzyl-2-(3-hydroxyimino-pyrrolidin-1-yl)-2-oxo-ethyl]-amide,
5-Chloro-1H-indole-2-carboxylic acid [2-(cis-3,4-dihydroxy-pyrrolidin-1-yl)-2-oxo-ethyl]-amide,
5-Chloro-1H-indole-2-carboxylic acid [2-((3S,4S)-dihydroxy-pyrrolidin-1-yl)-2-oxo-ethyl]-amide,
5-Chloro-1H-indole-2-carboxylic acid [(1S)-benzyl-2-(cis-3,4-dihydroxy-pyrrolidin-1-yl)-2-oxo-ethyl]-amide,
5-Chloro-1H-indole-2-carboxylic acid [2-(1,1-dioxo-thiazolidin-3-yl)-2-oxo-ethyl]-amide,
5-Chloro-1H-indole-2-carboxylic acid (2-oxo-2-thiazolidin-3-yl-ethyl)-amide,
5-Chloro-1H-indole-2-carboxylic acid [(1S)-(4-fluoro-benzyl)-2-(4-hydroxy-piperidin-1-yl)-2-oxo-ethyl]-amide,
5-Chloro-1H-indole-2-carboxylic acid [(1S)-benzyl-2-((3RS)-hydroxy-piperidin-1-yl)-2-oxo-ethyl]-amide,
5-Chloro-1H-indole-2-carboxylic acid [2-oxo-2-((1RS)-oxo-1-thiazolidin-3-yl)-ethyl]-amide,
5-Chloro-1H-indole-2-carboxylic acid [(1S)-(2-fluoro-benzyl)-2-(4-hydroxy-piperidin-1-yl)-2-oxo-ethyl]-amide,
5-Chloro-1H-indole-2-carboxylic acid [(1S)-benzyl-2-((3S,4S)-dihydroxy-pyrrolidin-1-yl)-2-oxo-ethyl]-amide,
5-Chloro-1H-indole-2-carboxylic acid [(1S)-benzyl-2-(3-hydroxy-azetidin-1-yl)-2-oxo-ethyl]-amide,
5-Chloro-1H-indole-2-carboxylic acid [(1S)-benzyl-2-(3-hydroxyimino-azetidin-1-yl)-2-oxo-ethyl]-amide,
5-Chloro-1H-indole-2-carboxylic acid [(1S)-benzyl-2-(4-hydroxyimino-piperidin-1-yl)-2-oxo-ethyl]-amide, and
5-Chloro-1H-indole-2-carboxylic acid [1-benzyl-2-(3-hydroxypyrrolidin-1-yl)-2-oxo-ethyl]amide.

In a further embodiment, the method comprises administering to a mammal, including a human, an effective amount of another group of particularly preferred compounds within the especially preferred compounds of Formula IA, consisting of the compounds wherein:

$R^{12}$ is H; and

$R^{15}$ is thiazolidin-3-yl, 1-oxo-thiazolidin-3-yl, 1,1-dioxo-thiazolidin-3-yl or oxazolidin-3-yl or said $R^{15}$ substituents optionally mono- or di-substituted independently with carboxy, $(C_1\text{-}C_5)$alkoxycarbonyl, hydroxy$(C_1\text{-}C_3)$alkyl, amino $(C_1\text{-}C_3)$alkyl, mono-N- or di-N,N-$(C_1\text{-}C_3)$alkylamino$(C_1\text{-}C_3)$alkyl or

$R^{15}$ is mono- or di-substituted pyrrolidin-1-yl wherein said substituents are independently carboxy, $(C_1\text{-}C_5)$alkoxycarbonyl, $(C_1\text{-}C_5)$alkoxy, hydroxy, hydroxy$(C_1\text{-}C_3)$alkyl, amino, amino$(C_1\text{-}C_3)$alkyl, mono-N- or di-N,N-$(C_1\text{-}C_3)$alkylamino$(C_1\text{-}C_3)$alkyl or mono-N- or di-N,N-$(C_1\text{-}C_4)$alkylamino, and

the $R^{15}$ rings are optionally additionally independently disubstituted with $(C_1\text{-}C_5)$alkyl.

In a further embodiment, the method comprises administering to a mammal, including a human, an effective amount of another group of particularly preferred compounds within the above particularly preferred compounds of Formula IA, consisting of the compounds wherein $R^1$ is 5-chloro, $R^8$ and $R^9$ are both H; and $R^{15}$ is selected from the group consisting of cis-3,4-dihydroxy-pyrrolidin-1-yl, (3S,4S)-dihydroxy-pyrrolidin-1-yl, 1,1-dioxo-thiazolidin-3-yl, thiazolidin-3-yl and 1-oxo-thiazolidin-3-yl.

In another embodiment, the method comprises administering to a mammal, including a human, an effective amount of another group of particularly preferred compounds within the especially preferred compounds of Formula IA, consisting of the compounds wherein:

$R^{15}$ is phenylmethyl, thien-2- or -3-ylmethyl wherein said $R^{15}$ rings are optionally mono- or di-substituted with fluoro; and

$R^{15}$ is thiazolidin-3-yl, 1-oxo-thiazolidin-3-yl, 1,1-dioxo-thiazolidin-3-yl or oxazolidin-3-yl or said $R^{15}$ substituents optionally mono- or di-substituted independently with carboxy or $(C_1\text{-}C_5)$alkoxycarbonyl, hydroxy$(C_1\text{-}C_3)$alkyl, amino$(C_1\text{-}C_3)$alkyl or mono-N- or di-N,N-$(C_1\text{-}C_3)$alkylamino$(C_1\text{-}C_3)$alkyl,

or $R^{15}$ is mono- or di-substituted azetidin-1-yl or mono- or di-substituted pyrrolidin-1-yl or mono- or di-substituted piperidin-1-yl wherein said substituents are independently carboxy, $(C_1\text{-}C_5)$alkoxycarbonyl, hydroxy$(C_1\text{-}C_3)$alkyl, amino$(C_1\text{-}C_3)$alkyl mono-N- or di-N,N-$(C_1\text{-}C_3)$alkylamino$(C_1\text{-}C_3)$alkyl, hydroxy, $(C_1\text{-}C_5)$alkoxy, amino, mono-N- or di-N,N-$(C_1\text{-}C_5)$alkylamino, oxo, hydroxyimino or $(C_1\text{-}C_5)$alkoxyimino; and

the $R^{15}$ rings are optionally additionally mono- or di-substituted independently with $(C_1\text{-}C_5)$alkyl.

In a further embodiment, the method comprises administering to a mammal, including a human, an effective amount of another group of particularly preferred compounds within the above particularly preferred compounds of Formula IA, consisting of the compounds wherein $R^1$ is 5-chloro, $R^8$ and $R^9$ are H, and wherein

a.

$R^{12}$ is 4-fluorobenzyl;
$R^{15}$ is 4-hydroxypiperidin-1-yl; and
the stereochemistry of carbon (a) is (S);

b.

$R^{12}$ is benzyl;
$R^{15}$ is 3-hydroxypiperidin-1-yl; and
the stereochemistry of carbon (a) is (S);

c.

$R^{12}$ is benzyl;
$R^{15}$ is cis-3,4-dihydroxy-pyrrolidin-1-yl; and
the stereochemistry of carbon (a) is S;

d.

$R^{12}$ is benzyl;
$R^{15}$ is 3-hydroxyimino-pyrrolidin-1-yl; and
the stereochemistry of carbon (a) is (S);

e.

$R^{12}$ is 2-fluorobenzyl;
$R^{15}$ is 4-hydroxypiperidin-1-yl; and
the stereochemistry of carbon (a) is (S);

f.

$R^{12}$ is benzyl;
$R^{15}$ is (3S,4S)-dihydroxy-pyrrolidin-1-yl; and
the stereochemistry of carbon (a) is (S);

g.

$R^{12}$ is benzyl;
$R^{15}$ is 3-hydroxy-azetidin-1-yl; and
the stereochemistry of carbon (a) is (S);

h.

$R^{12}$ is benzyl;
$R^{15}$ is 3-hydroxyimino-azetidin-1-yl; and
the stereochemistry of carbon (a) is (S); and

i.

$R^{12}$ is benzyl;
$R^{15}$ is 4-hydroxyimino-piperidin-1-yl; and
the stereochemistry of carbon (a) is (S).

The invention also relates to a method of inhibiting tumor growth in a mammal, comprising administering to the mammal an effective amount of a compound of Formula I or Formula IA, or any of the preferred, especially preferred, or particularly preferred compounds described above, wherein the tumor growth is dependent on glycogen phosphorylase activity.

This invention also relates to a method of inhibiting tumor growth in a mammal, including a human, comprising administering to the mammal an effective amount of a compound which is a glycogen phosphorylase inhibitor of Formula II having the structure:

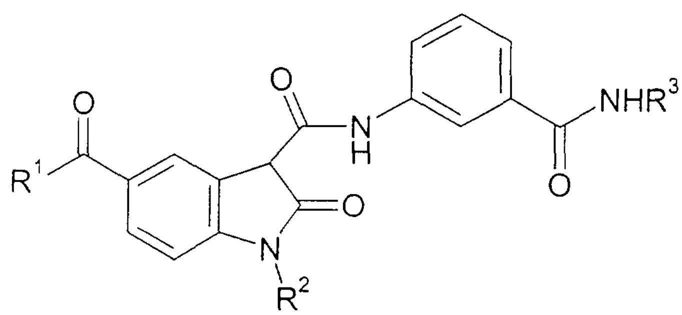

Formula II

prodrugs thereof and pharmaceutically acceptable salts of the compounds of Formula II and said prodrugs wherein in Formula II

$R^1$ is $(C_1\text{-}C_4)$alkyl, $(C_3\text{-}C_7)$cycloalkyl, phenyl or phenyl independently substituted with up to three of $(C_1\text{-}C_4)$alkyl, $(C_1\text{-}C_4)$alkoxy or halogen;
$R^2$ is $(C_1\text{-}C_4)$alkyl optionally substituted with up to three fluoro atoms; and
$R^3$ is $(C_3\text{-}C_7)$cycloalkyl, phenyl, phenyl substituted at the para position with $(C_1\text{-}C_4)$alkyl, halo or trifluoromethyl,

phenyl substituted at the meta position with fluoro, or phenyl substituted at the ortho position with fluoro.

In an embodiment, the method comprises administering to the mammal an effective amount of a compound of Formula II wherein $R^1$ is phenyl, $R^2$ is ethyl and $R^3$ is phenyl, phenyl substituted at the para position with ($C_1$-$C_4$) alkyl, halo or trifluoromethyl, phenyl substituted at the meta position with fluoro, or phenyl substituted at the ortho position with fluoro. In another embodiment, the method comprises administering to the mammal an effective amount of a compound of Formula II wherein $R^1$ is methyl and $R^2$ is ethyl.

This invention also relates to a method of inhibiting tumor growth in a mammal, including a human, comprising administering to the mammal an effective amount of a compound which is a glycogen phosphorylase inhibitor as disclosed by any of the patent applications WO 95/24391, WO 97/09040, WO 98/40353, WO 98/50359, WO 97/37901, EP-0884050, as well as the compounds disclosed by U.S. Patent No. 4,786,641.

This invention also relates to a method of inhibiting tumor growth in a mammal, including a human, comprising administering to the mammal an effective amount of a compound which is a glycogen phosphorylase inhibitor of Formula III having the structure:

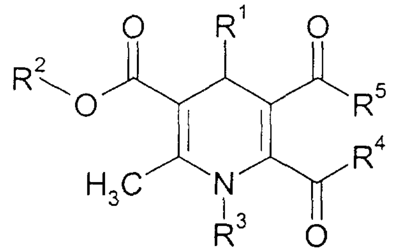

Formula III

wherein in Formula III, $R^1$ is phenyl, naphthyl, thenyl, pyridyl, chromenyl or thiochromenyl, substituted with up to two of halo, nitro, cyano, alkyl, ($C_1$-$C_6$)alkoxy and ($C_1$-$C_6$)alkylthio, ($C_1$-$C_3$)mono-, di-, or tri-fluoroalkyl or ($C_1$-$C_3$)mono-, di-, or tri-fluoroalkoxy; $R^2$ is ($C_1$-$C_8$)alkyl or ($C_1$-$C_8$)cycloalkyl, wherein the alkyl or cycloalkyl optionally contains up to one oxygen or sulfur atom and optionally is substituted with halo, phenyl, cyano, hydroxy, amino, ($C_1$-$C_3$)alkylamino or di($C_1$-$C_3$)alkylamino, or benzylmethylamino; $R^3$ is ($C_1$-$C_6$)alkyl or ($C_1$-$C_6$)cycloalkyl wherein the alkyl or alkylamino optionally contains up to one oxygen or sulfur atom and optionally is substituted with halo, phenyl, hydroxy, amino, morpholino, carboxy or ($C_1$-$C_4$)alkoxycarbonyl; and wherein $R^4$ and $R^5$ are each hydroxy or together represent —O—.

This invention also relates to a method of inhibiting tumor growth in a mammal, including a human, comprising administering to the mammal an effective amount of a compound which is a glycogen phosphorylase inhibitor of Formula IV having the structure:

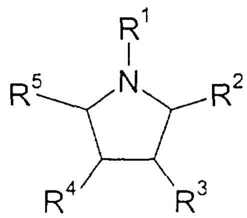

Formula IV

wherein in Formula IV, $R^1$ is H, ($C_1$-$C_6$)alkyl, ($C_1$-$C_6$)hydroxyalkyl, acetyl, allyl, ($C_1$-$C_6$)cycloalkyl, ($C_1$-$C_6$)cycloalkyl ($C_1$-$C_3$)alkyl, or ($C_1$-$C_6$)aminoalkyl, wherein the alkyl, cycloalkyl or cycloalkyl-alkyl is optionally substituted with up to three halo, up to two hydroxy on adjacent carbon atoms, or 2-phthalimidyl, wherein each of $R^2$, $R^3$ and $R^4$ is independently hydroxy, benzyloxy, benzyloxymethyl, ($C_1$-$C_3$)hydroxyalkyl, wherein $R^5$ is hydrogen.

This invention also relates to compounds of Formula IV, wherein $R^1$ is hydrogen, $R^2$ is $C(O)R^6$, $CH(OH)R^6$, $CHXR^6$ where X is halo, $CH(NH_2)R^6$ or $CHSR^6$, where $R^6$ is ($C_1$-$C_{14}$)alkyl (straight-chained or branched) optionally substituted with ($C_3$-$C_7$)cycloalkyl, ($C_1$-$C_6$)alkoxy, phenoxy, perhalomethyl, halogen, or phenyl; and wherein $R^3$ and $R^4$ are each independently hydroxy, halogen, amino or mercapto.

This invention also relates to a method of inhibiting tumor growth in a mammal, including a human, comprising administering to the mammal an effective amount of a compound which is a glycogen phosphorylase inhibitor of Formula IV and Formula V, wherein the compound of Formula V has the structure:

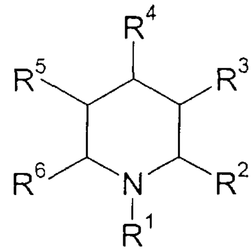

**Formula V**

wherein in this invention, the substituents of Formula IV and of Formula V are defined as follows:

$R^1$ is H, a monosaccharide moiety or $(C_1-C_8)$alkyl, wherein $R^1$ is optionally substituted with one or more of hydroxy, hydroxyalkyl, halogen, amino, $(C_1-C_8)$alkylamino, di-$(C_1-C_8)$alkyl-amino, tri$(C_1-C_8)$alkylammonium, nitro, formyl, carboxy, carboxy$(C_1-C_8)$alkyl, $(C_1-C_8)$alkylthio, $(C_1-C_8)$alkenyl, phenyl and $(C_1-C_8)$alkylphenyl, or $R^1$ together with the adjacent nitrogen atom from the pyrrolidinyl nucleus of Formula IV or the piperidine nucleus of Formula V represents a quaternary ammonium base ion containing two $(C_1-C_8)$alkyl groups optionally substituted as alkyl immediately above; wherein $R^2$, $R^3$, $R^4$ and $R^6$ are each independently H, hydroxy, hydroxyalkyl, halogen, amino, $(C_1-C_8)$alkylamino, $(C_1-C_8)$acylamino, N,N-di-$(C_1-C_8)$alkylamino, N,N,N-tri-$(C_1-C_8)$alkylammonium, nitro, formyl, carboxy, benzoxy, mercapto, $(C_1-C_8)$alkylthio, $(C_1-C_8)$alkenyl, phenyl and $(C_1-C_8)$alkylphenyl; and $R^5$ is phenyl or methyl optionally substituted with one or more of hydroxy, hydroxyalkyl, halogen, amino, $(C_1-C_8)$alkylamino, di-$(C_1-C_8)$alkyl-amino, tri$(C_1-C_8)$alkylammonium, nitro, formyl, carboxy, carboxy$(C_1-C_8)$alkyl, $(C_1-C_8)$alkylthio, $(C_1-C_8)$alkenyl, phenyl and $(C_1-C_8)$alkylphenyl, provided that the compound contains at least two free or protected hydroxy groups.

The invention also relates to a method of inhibiting tumor growth in a mammal comprising administering an effective amount of a compound of Formula I or Formula IA, or any of the preferred, especially preferred, or particularly preferred compounds described above, wherein the tumor is a colorectal, lung or breast tumor. In an embodiment of the invention, the tumor is an adenocarcinoma.

The invention also relates to a method of inhibiting tumor growth in a mammal, comprising administering to the mammal an effective amount of a compound which is a glycogen phosphorylase inhibitor of Formula I or Formula IA, or any of the preferred, especially preferred, or particularly preferred compounds described above, wherein the compound is administered in a pharmaceutical composition comprising the compound and a pharmaceutically acceptable carrier.

The invention also relates to a method of inhibiting tumor growth in a mammal comprising administering to said mammal an effective amount of a compound which is a glycogen phosphorylase inhibitor, wherein the inhibition of recombinant human liver glycogen phosphorylase ("HLGPa") by the compound, in the presence of 7.5 mM glucose, is characterized by an $IC_{50}$ having a value less than 100 nanomolar.

The $IC_{50}$ value is defined as the concentration of the subject compound required to produce an enzyme activity of 50%, compared to 100% enzyme activity in the absence of the compound and 0% activity when the enzyme is fully inhibited, e.g., by caffeine. In this invention, enzyme activity is determined by measurement of the reverse activity of HLGPa, i.e., by measuring the release of phosphate due to the incorporation of glucose-1-phosphate into glycogen, where the amount of phosphate released is determined using the spectrophotometric method and experimental conditions described hereinbelow. Because the determined $IC_{50}$ may vary from one experiment to another, it is to be understood that the $IC_{50}$ value reflects the average value of the $IC_{50}$ determined from multiple repetitions of dose-response experiments using a particular compound (e.g., ten or more $IC_{50}$ determinations wherein the observed maximal and minimum activities are as expected).

The invention also relates to a pharmaceutical composition for the inhibition of tumor growth in a mammal comprising an effective amount of a glycogen phosphorylase inhibitor, or a pharmaceutically acceptable salt or prodrug thereof, in combination with a pharmaceutically acceptable carrier, wherein the glycogen phosphorylase inhibitor is a compound of Formula I or Formula IA, or any of the preferred, especially preferred, or particularly preferred compounds of Formula I or Formula IA, or a pharmaceutically acceptable salt or prodrug thereof.

The glycogen phosphorylase inhibitor of Formulas I and IA are employed separately or in combination to inhibit tumor growth or treat cancer. Accordingly, this invention relates to a method of inhibiting tumor growth comprising administering a compound of Formula I or Formula IA together with a compound which inhibits a protein tyrosine kinase,

or alternatively, together with a compound which inhibits farnesylation of the Ras oncogene.

Mutated, oncogenic forms of Ras are frequently found in many human cancers, most notably in more than 50% of colon and pancreatic carcinomas (Kohl et al., Science, Vol. 260, 1834 to 1837, 1993). Oncogenes frequently encode protein components of signal transduction pathways which lead to stimulation of cell growth and mitogenesis. Oncogene expression in cultured cells leads to cellular transformation, characterized by the ability of cells to grow in soft agar and the growth of cells as dense foci lacking the contact inhibition exhibited by non-transformed cells. Mutation and/or overexpression of certain oncogenes is frequently associated with human cancer. To acquire transforming potential, the precursor of the Ras oncoprotein must undergo farnesylation of the cysteine residue located in a carboxyl-terminal tetrapeptide. Inhibitors of the enzyme that catalyzes this modification, farnesyl protein transferase, have therefore been suggested as agents to combat tumors in which Ras contributes to transformation.

The invention also relates to a method for the treatment of a hyperproliferative disorder in a mammal which comprises administering to the mammal a therapeutically effective amount of a compound of Formula I or Formula IA, or a pharmaceutically acceptable salt, prodrug or hydrate thereof, in combination with a protein tyrosine kinase inhibitor or Ras farnesylation inhibitor, and further relates to pharmaceutical compositions comprising the compound and the Ras farnesylation inhibitor and a pharmaceutically acceptable carrier. In one embodiment, said method or pharmaceutical composition is for the treatment of cancer such as lung, squamous cell, gastric, breast or colorectal cancer. In another embodiment, the method or pharmaceutical composition is for the treatment of cancer of the brain bladder, pancreas, head, neck, kidney (in this document, this includes proliferative glomerulonephritis), ovary, cervix, prostate, esophagus, or thyroid. In another embodiment, said pharmaceutical composition is for the treatment of a non-cancerous hyperproliferative disorder such as benign hyperplasia of the skin (e.g., psoriasis) or prostate (e.g., benign prostatic hypertrophy (BPH)).

The invention also relates to a pharmaceutical composition for treating a disease related to vasculogenesis or angiogenesis in a mammal which comprises a therapeutically effective amount of a compound of Formula I or Formula IA or a pharmaceutically acceptable salt, prodrug or hydrate thereof in combination with an angiogenesis inhibitor, and a pharmaceutically acceptable carrier. In one embodiment, said method or pharmaceutical composition is for treating a disease selected from the group consisting of tumor angiogenesis, chronic inflammatory disease such as rheumatoid arthritis, atherosclerosis, skin diseases such as psoriasis, eczema, and scleroderma, diabetes, diabetic retinopathy, retinopathy of prematurity, age-related macular degeneration, hemangioma, glioma, melanoma, Kaposi's sarcoma and ovarian, breast, lung, pancreatic, prostate, colon and epidermoid cancer.

The invention also relates to a method for the treatment of a hyperproliferative disorder in a mammal which comprises administering to said mammal a therapeutically effective amount of a compound of Formula I or Formula IA, or a pharmaceutically acceptable salt, prodrug or hydrate thereof, in combination with an anti-tumor agent selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, anti-hormones, and anti-androgens. The invention also relates to a pharmaceutical composition comprising a compound of Formula I or IA and any of the antitumor agents described above, and a pharmaceutically acceptable carrier.

Patients that can be treated with a compounds of Formula I or Formula IA, and the pharmaceutically acceptable salts, prodrugs and hydrates of said compounds, according to the methods of this invention include, for example, patients that have been diagnosed as having psoriasis, BPH, lung cancer, bone cancer, pancreatic cancer, skin cancer, cancer of the head and neck, cutaneous or intraocular melanoma, uterine cancer, ovarian cancer, rectal cancer, cancer of the anal region, stomach cancer, colon cancer, breast cancer, gynecologic tumors (e.g., uterine sarcomas, carcinoma of the fallopian tubes, carcinoma of the endometrium, carcinoma of the cervix, carcinoma of the vagina or carcinoma of the vulva), Hodgkin's disease, cancer of the esophagus, cancer of the small intestine, cancer of the endocrine system (e.g., cancer of the thyroid, parathyroid or adrenal glands), sarcomas of soft tissues, cancer of the urethra, cancer of the penis, prostate cancer, chronic or acute leukemia, solid tumors of childhood, lymphocytic lymphomas, cancer of the bladder, cancer of the kidney or ureter (e.g., renal cell carcinoma, carcinoma of the renal pelvis), or neoplasms of the central nervous system (e.g., primary CNS lymphoma, spinal axis tumors, brain stem gliomas or pituitary adenomas).

This invention also relates to a pharmaceutical composition for inhibiting abnormal cell growth in a mammal which comprises an amount of a compound of Formula I or Formula IA, or a pharmaceutically acceptable salt or solvate or prodrug thereof, in combination with an amount of a chemotherapeutic, wherein the amounts of the compound, salt, solvate, or prodrug, and of the chemotherapeutic are together effective in inhibiting abnormal cell growth. Many chemotherapeutics are presently known in the art. In one embodiment, the chemotherapeutic is selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, anti-hormones, *e.g.* anti-androgens.

This invention further relates to a method for inhibiting abnormal cell growth in a mammal which method comprises administering to the mammal an amount of a compound of Formula I or Formula IA, or a pharmaceutically acceptable salt or solvate or prodrug thereof, in combination with radiation therapy, wherein the amount of the compound, salt, solvate or prodrug is in combination with the radiation therapy effective in inhibiting abnormal cell growth in the mammal. Techniques for administering radiation therapy are known in the art, and these techniques can be used in the combination therapy described herein. The administration of the compound of the invention in this combination therapy can be determined using methods well-known in the art.

It is believed that the compounds of Formula I and Formula IA can render abnormal cells more sensitive to treatment with radiation for purposes of killing and/or inhibiting the growth of such cells. Accordingly, this invention further relates to a method for sensitizing abnormal cells in a mammal to treatment with radiation which comprises administering to the mammal an amount of a compound of Formula I or Formula IA or pharmaceutically acceptable salt, prodrug or solvate thereof, which amount is effective in sensitizing abnormal cells to treatment with radiation. The amount of the compound, salt, or solvate in this method can be determined according to the means for ascertaining effective amounts of such compounds which are well-known in the art.

This invention also relates to a method of and to a pharmaceutical composition for inhibiting abnormal cell growth in a mammal which comprises an amount of a compound of Formula I or Formula IA, a pharmaceutically acceptable salt or solvate thereof, a prodrug thereof, or an isotopically-labelled derivative thereof, and an amount of one or more substances selected from anti-angiogenesis agents, signal transduction inhibitors, and antiproliferative agents.

Anti-angiogenesis agents, such as MMP-2 (matrix-metalloproteinase 2) inhibitors, MMP-9 (matrix-metalloproteinase 9) inhibitors, and COX-II (cyclooxygenase II) inhibitors, can be used in conjunction with a compound of Formula I or Formula IA and pharmaceutical compositions described herein. Examples of useful COX-II inhibitors include CELEBREX™ (celecoxib), valdecoxib, and rofecoxib. Examples of useful matrix metalloproteinase inhibitors are described in WO 96/33172 (published October 24, 1996), WO 96/27583 (published March 7, 1996), European Patent Application No. 97304971.1 (filed July 8, 1997), European Patent Application No. 99308617.2 (filed October 29, 1999), WO 98/07697 (published February 26, 1998), WO 98/03516 (published January 29, 1998), WO 98/34918 (published August 13, 1998), WO 98/34915 (published August 13, 1998), WO 98/33768 (published August 6, 1998), WO 98/30566 (published July 16, 1998), European Patent Publication 606,046 (published July 13, 1994), European Patent Publication 931,788 (published July 28, 1999), WO 90/05719 (published May 331, 1990), WO 99/52910 (published October 21, 1999), WO 99/52889 (published October 21, 1999), WO 99/29667 (published June 17, 1999), PCT International Application No. PCT/IB98/01113 (filed July 21, 1998), European Patent Application No. 99302232.1 (filed March 25, 1999), Great Britain patent application number 9912961.1 (filed June 3, 1999), United States Provisional Application No. 60/148,464 (filed August 12, 1999), United States Patent 5,863,949 (issued January 26, 1999), United States Patent 5,861,510 (issued January 19, 1999), and European Patent Publication 780,386 (published June 25, 1997), all of which are incorporated herein in their entireties by reference. Preferred MMP-2 and MMP-9 inhibitors are those that have little or no activity inhibiting MMP-1. More preferred, are those that selectively inhibit MMP-2 and/or MMP-9 relative to the other matrix-metalloproteinases (*i.e.* MMP-1, MMP-3, MMP-4, MMP-5, MMP-6, MMP-7, MMP-8, MMP-10, MMP-11, MMP-12, and MMP-13).

Some specific examples of MMP inhibitors useful in the present invention are AG-3340, RO 32-3555, RS 13-0830, and the compounds recited in the following list:

3-[[4-(4-fluoro-phenoxy)-benzenesulfonyl]-(1-hydroxycarbamoyl-cyclopentyl)-amino]-propionic acid;
3-exo-3-[4-(4-fluoro-phenoxy)-benzenesulfonylamino]-8-oxa-bicyclo[3.2.1]octane-3-carboxylic acid hydroxyamide;
(2R, 3R) 1-[4-(2-chloro-4-fluoro-benzyloxy)-benzenesulfonyl]-3-hydroxy-3-methyl-piperidine-2-carboxylic acid hydroxyamide;
4-[4-(4-fluoro-phenoxy)-benzenesulfonylamino]-tetrahydro-pyran-4-carboxylic acid hydroxyamide;
3-[[4-(4-fluoro-phenoxy)-benzenesulfonyl]-(1-hydroxycarbamoyl-cyclobutyl)-amino]-propionic acid;
4-[4-(4-chloro-phenoxy)-benzenesulfonylamino]-tetrahydro-pyran-4-carboxylic acid hydroxyamide;
(R) 3-[4-(4-chloro-phenoxy)-benzenesulfonylamino]-tetrahydro-pyran-3-carboxylic acid hydroxyamide;
(2R, 3R) 1 -[4-(4-fluoro-2-methyl-benzyloxy)-benzenesulfonyl]-3-hydroxy-3-methyl-piperidine-2-carboxylic acid hydroxyamide;
3-[[4-(4-fluoro-phenoxy)-benzenesulfonyl]-(1-hydroxycarbamoyl-1-methyl-ethyl)-amino]-propionic acid;
3-[[4-(4-fluoro-phenoxy)-benzenesulfonyl]-(4-hydroxycarbamoyl-tetrahydro-pyran-4-yl)-amino]-propionic acid;
3-exo-3-[4-(4-chloro-phenoxy)-benzenesulfonylamino]-8-oxa-bicyclo[3.2.1]octane-3-carboxylic acid hydroxyamide;
3-endo-3-[4-(4-fluoro-phenoxy)-benzenesulfonylamino]-8-oxa-bicyclo[3.2.1]octane-3-carboxylic acid hydroxyamide; and
(R) 3-[4-(4-fluoro-phenoxy)-benzenesulfonylamino]-tetrahydro-furan-3-carboxylic acid hydroxyamide;
and pharmaceutically acceptable salts and solvates of said compounds.

Other anti-angiogenesis agents, including other COX-II inhibitors and other MMP inhibitors, can also be used

in the present invention.

A compound of Formula I or Formula IA, can also be used with signal transduction inhibitors, such as agents that can inhibit EGFR (epidermal growth factor receptor) responses, such as EGFR antibodies, EGF antibodies, and molecules that are EGFR inhibitors; VEGF (vascular endothelial growth factor) inhibitors, such as VEGF receptor inhibitors and molecules that can inhibit VEGF; and erbB2 receptor inhibitors, such as organic molecules or antibodies that bind to the erbB2 receptor, for example, HERCEPTIN™ (Genentech, Inc. of South San Francisco, California, USA).

EGFR inhibitors are described in, for example in WO 95/19970 (published July 27, 1995), WO 98/14451 (published April 9, 1998), WO 98/02434 (published January 22, 1998), and United States Patent 5,747,498 (issued May 5, 1998), and such substances can be used in the present invention as described herein. EGFR-inhibiting agents include, but are not limited to, the monoclonal antibodies C225 and anti-EGFR 22Mab (ImClone Systems Incorporated of New York, New York, USA), the compounds ZD-1839 (AstraZeneca), BIBX-1382 (Boehringer Ingelheim), MDX-447 (Medarex Inc. of Annandale, New Jersey, USA), and OLX-103 (Merck & Co. of Whitehouse Station, New Jersey, USA), VRCTC-310 (Ventech Research) and EGF fusion toxin (Seragen Inc. of Hopkinton, Massachusetts). These and other EGFR-inhibiting agents can be used in the present invention.

VEGF inhibitors, for example SU-5416 and SU-6668 (Sugen Inc. of South San Francisco, California, USA), can also be combined with the compound of the present invention. VEGF inhibitors are described in, for example in WO 99/24440 (published May 20, 1999), PCT International Application PCT/IB99/00797 (filed May 3, 1999), in WO 95/21613 (published August 17, 1995), WO 99/61422 (published December 2, 1999), United States Patent 5,834,504 (issued November 10, 1998), WO 98/50356 (published November 12, 1998), United States Patent 5,883,113 (issued March 16, 1999), United States Patent 5,886,020 (issued March 23, 1999), United States Patent 5,792,783 (issued August 11, 1998), WO 99/10349 (published March 4, 1999), WO 97/32856 (published September 12, 1997), WO 97/22596 (published June 26, 1997), WO 98/54093 (published December 3, 1998), WO 98/02438 (published January 22, 1998), WO 99/16755 (published April 8, 1999), and WO 98/02437 (published January 22, 1998), all of which are incorporated herein in their entireties by reference. Other examples of some specific VEGF inhibitors useful in the present invention are IM862 (Cytran Inc. of Kirkland, Washington, USA); anti-VEGF monoclonal antibody of Genentech, Inc. of South San Francisco, California; and angiozyme, a synthetic ribozyme from Ribozyme (Boulder, Colorado) and Chiron (Emeryville, California). These and other VEGF inhibitors can be used in the present invention as described herein.

ErbB2 receptor inhibitors, such as GW-282974 (Glaxo Wellcome plc), and the monoclonal antibodies AR-209 (Aronex Pharmaceuticals Inc. of The Woodlands, Texas, USA) and 2B-1 (Chiron), can furthermore be combined with the compound of the invention, for example those indicated in WO 98/02434 (published January 22, 1998), WO 99/35146 (published July 15, 1999), WO 99/35132 (published July 15, 1999), WO 98/02437 (published January 22, 1998), WO 97/13760 (published April 17, 1997), WO 95/19970 (published July 27, 1995), United States Patent 5,587,458 (issued December 24, 1996), and United States Patent 5,877,305 (issued March 2, 1999), which are all hereby incorporated herein in their entireties by reference. ErbB2 receptor inhibitors useful in the present invention are also described in United States Provisional Application No. 60/117,341, filed January 27, 1999, and in United States Provisional Application No. 60/117,346, filed January 27, 1999, both of which are incorporated in their entireties herein by reference. The erbB2 receptor inhibitor compounds and substance described in the aforementioned PCT applications, U.S. patents, and U.S. provisional applications, as well as other compounds and substances that inhibit the erbB2 receptor, can be used with the compound of the present invention in accordance with the present invention.

The compound of the invention can also be used with other agents useful in treating abnormal cell growth or cancer, including, but not limited to, agents capable of enhancing antitumor immune responses, such as CTLA4 (cytotoxic lymphocyte antigen 4) antibodies, and other agents capable of blocking CTLA4; and anti-proliferative agents such as other farnesyl protein transferase inhibitors, and the like. Specific CTLA4 antibodies that can be used in the present invention include those described in United States Provisional Application 60/113,647 (filed December 23, 1998) which is incorporated by reference in its entirety, however other CTLA4 antibodies can be used in the present invention.

The subject invention also includes isotopically-labelled compounds, which are identical to those recited in Formula I and Formula IA, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine and chlorine, such as $^{2}H$, $^{3}H$, $^{13}C$, $^{14}C$, $^{15}N$, $^{18}O$, $^{17}O$, $^{31}P$, $^{32}P$, $^{35}S$, $^{18}F$, and $^{36}Cl$, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically-labelled compounds of the present invention, for example those into which radioactive isotopes such as $^{3}H$ and $^{14}C$ are incorporated, are useful in drug and/or substrate tissue distribution assays. Tritiated, i.e., $^{3}H$, and carbon-14, i.e., $^{14}C$, isotopes are particularly preferred for their ease of preparation and detectability. Further, substitution with heavier isotopes such as deuterium, i.e., $^{2}H$, can afford certain therapeutic advantages resulting from

greater metabolic stability, for example increased *in vivo* half-life or reduced dosage requirements and, hence, may be preferred in some circumstances. Isotopically labelled compounds of Formula I or Formula IA of this invention and prodrugs thereof can generally be prepared by carrying out the procedures disclosed in the Schemes and/or in the Examples below, by substituting a readily available isotopically labelled reagent for a non-isotopically labelled reagent.

The term "compound of the invention" as used herein includes not only the compound of Formula I or Formula IA in its broadest definition, but also the preferred, especially preferred and particularly preferred embodiments of the compounds of Formula I and Formula IA.

The term "compound of Formula I or Formula IA" as used in the description of said compound in combination with other antiproliferative and anti-tumor agents as described hereinabove, is in that context meant to include not only a compound of Formula I or Formula IA in its broadest definition, but also the preferred, especially preferred and particularly preferred embodiments of the compounds of Formula I and Formula IA.

The terms "tumor growth" and "abnormal cell growth" as used herein, refer to cell growth that is independent of normal regulatory mechanisms (e.g., loss of contact inhibition). This includes, but is not limited to, the abnormal growth of: (1) tumor cells (tumors), both benign and malignant, due to direct expression of an oncogene or as a result of oncogenic mutation in another gene, or as a result of aberrant cell cycle regulation. Examples of malignancies particularly associated with glycogen storage are colorectal and lung cancer, particularly adenocarcinomas of the colon and lung, stomach and gastric cancer, particularly gastric carcinomas. Other cancers which may be treated using a glycogen phosphorylase inhibitor include NSCLC (non small cell lung cancer), bone cancer, pancreatic cancer, skin cancer, cancer of the head and neck, cutaneous or intraocular melanoma, uterine cancer, ovarian cancer, cancer of the anal region, gynecologic tumors (e.g., uterine sarcomas, carcinoma of the fallopian tubes, carcinoma of the endometrium, carcinoma of the cervix, carcinoma of the vagina or carcinoma of the vulva), Hodgkin's Disease, cancer of the esophagus, cancer of the small intestine, cancer of the endocrine system (e.g., cancer of the thyroid, parathyroid or adrenal glands), sarcomas of soft tissues, cancer of the urethra, cancer of the penis, prostate cancer, chronic or acute leukemia, solid tumors of childhood, lymphocytic lymphomas, cancer of the bladder, cancer of the kidney or ureter (e.g., renal cell carcinoma, carcinoma of the renal pelvis), pediatric malignancy, neoplasms of the central nervous system (e.g., primary CNS lymphoma, spinal axis tumors, brain stem gliomas or pituitary adenomas), neoplastic cutaneous diseases (e.g. psoriasis, mycoses fungoides), or Barrett's esophagus (pre-malignant syndrome).

The term "treating", as used herein, unless otherwise indicated, means reversing, alleviating, inhibiting the progress of, or preventing the disorder or condition to which such term applies, or one or more symptoms of such disorder or condition. The term "treatment", as used herein, refers to the act of treating, as "treating" is defined immediately above.

The terms "therapeutically effective amount" and "effective amount" mean an amount of a compound or combination of compounds that ameliorates, attenuates, or eliminates one or more symptoms of a particular disease or condition or prevents or delays the onset of one or more symptoms of a particular disease or condition.

The term "patient" means animals, such as dogs, cats, cows, horses, sheep, and humans. Particularly preferred patients are mammals. The term patient includes males and females.

The term "pharmaceutically acceptable" means that the carrier, diluent, excipients, and/or salt must be compatible with the other ingredients of the formulation, and not deleterious to the patient. Examples of suitable pharmaceutical carriers and suitable pharmaceutical salts are further described below.

The term "glycogen phosphorylase inhibitor " or "GPI" refers to any substance or any combination of substances that reduces, retards, or eliminates the enzymatic action of glycogen phosphorylase. The currently known enzymatic action of glycogen phosphorylase is the degradation of glycogen by catalysis of the reversible reaction of a glycogen macromolecule and inorganic phosphate to glucose-1-phosphate and a glycogen macromolecule which is one glucosyl residue shorter than the original glycogen macromolecule (forward direction of glycogenolysis). The phrase "glycogen phosphorylase inhibitor" includes the stereoisomers, pharmaceutically acceptable salts, prodrugs, and pharmaceutically acceptable salts of the prodrugs of the glycogen phosphorylase inhibitor.

## Detailed Description of the Invention

All patents, patent publications, and literature references cited herein are hereby incorporated by reference. It is intended that reference to particular compounds herein be interpreted to mean that the pharmaceutically acceptable anionic or cationic salts and prodrugs of those compounds may also be employed.

Methods for making the glycogen phosphorylase inhibitors described herein are described in detail in U.S. Patent No. 5,952,322 and in WO 96/39384 and WO 96/39385, as well as in U.S. Patent No. 5,998,463, WO 95/24391, WO 97/09040, WO 98/40353, WO 98/50359, WO 97/37901, EP-0884050, and U.S. Patent No. 4,786,641, all of which are hereby incorporated by reference into this application.

By "halo" is meant chloro, bromo, iodo, or fluoro.

By "alkyl" is meant straight chain or branched saturated hydrocarbon. Exemplary of such alkyl groups (assuming the designated length encompasses the particular example) are methyl, ethyl, propyl, isopropyl, butyl, sec-butyl, tertiary butyl, pentyl, isopentyl, hexyl and isohexyl.

By "alkoxy" is meant straight chain or branched saturated alkyl bonded through an oxy. Exemplary of such alkoxy groups (assuming the designated length encompasses the particular example) are methoxy, ethoxy, propoxy, isopropoxy, butoxy, isobutoxy, tertiary butoxy, pentoxy, isopentoxy, hexoxy and isohexoxy.

The term "pharmaceutically acceptable salt(s)", as used herein, unless otherwise indicated, includes salts of acidic or basic groups that may be present in the compounds of Formula I or Formula IA. For example, pharmaceutically acceptable salts include sodium, calcium and potassium salts of carboxylic acid groups and hydrochloride salts of amino groups. Other pharmaceutically acceptable salts of amino groups are hydrobromide, sulfate, hydrogen sulfate, phosphate, hydrogen phosphate, dihydrogen phosphate, acetate, succinate, citrate, tartrate, lactate, mandelate, methanesulfonate (mesylate) and p-toluenesulfonate (tosylate) salts. The preparation of such salts is described below.

The expression "pharmaceutically-acceptable anionic salt" refers to nontoxic anionic salts containing anions such as (but not limited to) chloride, bromide, iodide, sulfate, bisulfate, phosphate, acetate, maleate, fumarate, oxalate, lactate, tartrate, citrate, gluconate, methanesulfonate and 4-toluene-sulfonate.

The expression "pharmaceutically-acceptable cationic salt" refers to nontoxic cationic salts such as (but not limited to) sodium, potassium, calcium, magnesium, ammonium or protonated benzathine (N,N'-dibenzylethylenediamine), choline, ethanolamine, diethanolamine, ethylenediamine, meglumine (N-methyl-glucamine), benethamine (N-benzylphenethylamine), piperazine or tromethamine (2-amino-2-hydroxymethyl-1,3-propanediol).

This invention relates to a method of inhibiting tumor growth in a mammal, comprising administering to the mammal an effective amount of a pharmaceutically acceptable salt of the compound of Formula I or Formula IA, or any of the preferred, especially preferred or particularly preferred compounds of Formula I or Formula IA, including pharmaceutically acceptable cationic salts and pharmaceutically acceptable anionic salts of the compounds of Formula I or Formula IA.

The expression "prodrug" refers to compounds that are drug precursors, which following administration, release the drug in vivo via some chemical or physiological process (e.g., a prodrug on being brought to the physiological pH is converted to the desired drug form). Exemplary prodrugs upon cleavage release the corresponding free acid, and such hydrolyzable ester-forming residues of the compounds of Formula I and Formula IA include but are not limited to carboxylic acid substituents (e.g., $R^{10}$ contains carboxy) wherein the free hydrogen is replaced by $(C_1-C_4)$alkyl $(C_2-C_{12})$alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-$(C_1-C_2)$alkylamino$(C_2-C_3)$alkyl (such as $\alpha$-dimethylaminoethyl), carbamoyl-$(C_1-C_2)$alkyl, N,N-di$(C_1-C_2)$alkylcarbamoyl-$(C_1-C_2)$alkyl and piperidino-, pyrrolidino- or morpholino$(C_2-C_3)$alkyl.

Other exemplary prodrugs release an alcohol of Formula I or Formula IA wherein the free hydrogen of the hydroxy substituent (e.g., $R^5$ is hydroxy) is replaced by $(C_1-C_6)$alkanoyloxymethyl, 1-($(C_1-C_6)$alkanoyloxy)ethyl, 1-methyl-1-($(C_1-C_6)$alkanoyloxy)ethyl, $(C_1-C_6)$alkoxycarbonyloxymethyl, N-$(C_1-C_6)$alkoxycarbonylaminomethyl, succinoyl, $(C_1-C_6)$alkanoyl, $\alpha$-amino$(C_1-C_4)$alkanoyl, arylacyl and $\alpha$-aminoacyl, or $\alpha$-aminoacyl-$\alpha$-aminoacyl wherein said $\alpha$-aminoacyl moieties are independently any of the naturally occurring L-amino acids found in proteins, $P(O)(OH)_2$, $-P(O)(O(C_1-C_6)alkyl)_2$ or glycosyl (the radical resulting from detachment of the hydroxyl of the hemiacetal of a carbohydrate).

Other exemplary prodrugs include but are not limited to derivatives of Formula I or Formula IA wherein $R^2$ is a free hydrogen which is replaced by R-carbonyl, RO-carbonyl, NRR'-carbonyl where R and R' are each independently ($(C_1-C_{10})$alkyl, $(C_3-C_7)$cycloalkyl, benzyl, or R-carbonyl is a natural $\alpha$-aminoacyl or natural $\alpha$-aminoacyl-natural $\alpha$-aminoacyl,-C(OH)C(O)OY wherein Y is H, $(C_1-C_6)$alkyl or benzyl, $-C(OY_0)Y_1$ wherein $Y_0$ is $(C_1-C_4)$ alkyl and $Y_1$ is ($(C_1-C_6)$alkyl, carboxy$(C_1-C_6)$alkyl, amino$(C_1-C_4)$alkyl or mono-N- or di-N,N-$(C_1-C_6)$alkylaminoalkyl, $-C(Y_2)Y_3$ wherein $Y_2$ is H or methyl and $Y_3$ is mono-N- or di-N,N-$(C_1-C_6)$alkylamino, morpholino, piperidin-1-yl or pyrrolidin-1-yl.

Other exemplary prodrugs include but are not limited to derivatives of Formula I or Formula IA bearing a hydrolyzable moiety at $R^3$, which release a compound of Formula I or Formula IA wherein $R^3$ is a free hydrogen on hydrolysis. Such hydrolyzable moieties at $R^3$ are/include 1-hydroxy$(C_1-C_6)$alkyl or 1-hydroxy-1-phenylmethyl.

Other exemplary prodrugs include cyclic structures such as compounds of Formula I or Formula IA wherein $R^2$ and $R^3$ are a common carbon, thus forming a five-membered ring. The linking carbon may be mono- or di-substituted independently with H, $(C_1-C_6)$alkyl, $(C_3-C_6)$cycloalkyl or phenyl. Alternatively, $R^3$ and $R^5$ may be taken together to form an oxazolidine ring and the number 2 carbon of the oxazolidine ring may be mono- or di-substituted independently with H, $(C_1-C_6)$alkyl, $(C_3-C_6)$cycloalkyl or phenyl.

Mammals treated according to the invention include but are not limited to humans. In one embodiment, the mammal is a companion animal, such as a dog or cat.

The chemist of ordinary skill will recognize that certain compounds of Formula I and Formula IA contain one

or more atoms which may be in a particular stereochemical or geometric configuration, giving rise to stereoisomers and configurational isomers. Examples of such atoms are the carbon atoms labelled "a" and "b" in Formula I (also referred to herein as "carbon (a)" or "carbon (b)" respectively), and the carbon atom labelled "a" in Formula 1A (also referred to herein as "carbon (a)" ). All such isomers and mixtures thereof are included in the method and composition of the invention. Hydrates of the compounds of Formula I and Formula IA are also included.

The compounds of Formula I and Formula IA have asymmetric carbon atoms and therefore are enantiomers or diastereomers. Diastereomeric mixtures can be separated into their individual diastereomers on the basis of their physical chemical differences by methods known per se., for example, by chromatography and/or fractional crystallization. Enantiomers can be separated by converting the enantiomeric mixture into a diastereomeric mixture by reaction with an appropriate optically active compound (e.g., alcohol), separating the diastereomers and converting (e.g., hydrolyzing) the individual diastereomers to the corresponding pure enantiomers. All such isomers, including diastereomers, enantiomers and mixtures thereof are considered as part of the method and composition of this invention. Use of any tautomers of compounds of Formula I and IA is also encompassed by the invention.

Although many compounds employed in this invention are not ionizable at physiological conditions, some of the compounds employed in this invention are ionizable at physiological conditions. Thus, for example some of the compounds employed in this invention are acidic and they form a salt with a pharmaceutically acceptable cation. All such salts are within the scope of the method and composition of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

In addition, some of the compounds employed in this invention are basic, and they form a salt with a pharmaceutically acceptable anion. All such salts are within the scope of the method and composition of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

In addition, use of any hydrates or solvates of compounds of Formula I or Formula IA is also within the scope of the invention.

Glycogen phosphorylase inhibition is readily determined by those skilled in the art according to standard assays. Methods for obtaining glycogen phosphorylases, and assays for determining glycogen phosphorylase inhibition are described below and are also well known in the art.

Glycogen phosphorylase from mammalian sources:

The three different purified glycogen phosphorylase (GP) isoenzymes from a human source, wherein glycogen phosphorylase is in the activated "a" state (referred to as glycogen phosphorylase a, or the abbreviation GPa), and referred to here as human liver glycogen phosphorylase a (HLGPa), human muscle glycogen phosphorylase a (HMGPa), and human brain glycogen phosphorylase a (HBGPa), can be obtained by the following procedures.

Expression and fermentation

The HLGP, and HMGP cDNAs are expressed from plasmid pKK233-2 (Pharmacia Biotech. Inc., Piscataway, New Jersey) in *E. coli* strain XL-1 Blue (Stratagene Cloning Systems, LaJolla, CA). The strain is inoculated into LB medium (consisting of 10 g tryptone, 5 g yeast extract, 5 g NaCl, and 1 mL 1N NaOH per liter) plus 100 mg/L ampicillin, 100 mg/L pyridoxine and 600 mg/L $MnCl_2$ and grown at 37°C to a cell density of $OD_{550}$= 1.0. At this point, the cells are induced with 1 mM isopropyl-1-thio-β-D-galactoside (IPTG). Three hours after induction the cells are harvested by centrifugation and cell pellets are frozen at -70°C until needed for purification.

The HBGP cDNA can be expressed by several methodologies, for example, by the method described by Crerar, et al. (J. Biol. Chem. 270:13748-13756). The method described by Crerar, et al. for the expression of HBGP is as follows: the HBGP cDNA can be expressed from plasmid pTACTAC in *E. Coli* strain 25A6. The strain is inoculated into LB medium (consisting of 10 g tryptone, 5 g yeast extract, 5 g NaCl, and 1 mL 1N NaOH per liter) plus 50 mg/L ampicillin and grown overnight, then resuspended in fresh LB medium plus 50 mg/L ampicillin, and reinoculated into a 40X volume of LB/amp media containing 250 μM isopropyl-1-thio-β-D-galactoside (IPTG), 0.5 mM pyridoxine and 3 mM mg/L and grown at 22°C for 48-50 hours. The cells can then be harvested by centrifugation and cell pellets are frozen at -70°C until needed for purification.

The HLGP cDNA is expressed from plasmid pBlueBac III (Invitrogen Corp., San Diego, CA) which is cotransfected with BaculoGold Linear Viral DNA (Pharmingen, San Diego, CA) into Sf9 cells. Recombinant virus is subsequently plaque-purified. For production of protein, Sf9 cells grown in serum-free medium are infected at an MOI of 0.5 and at a cell density of $2x10^6$ cells/mL. After growth for 72 hours at 27°C, cells are centrifuged, and the cell pellets frozen at -70°C until needed for purification.

Purification of Mammalian Glycogen Phosphorylase expressed in *E. coli*

The *E. coli* cells in pellets described above are resuspended in 25 mM β-glycerophosphate (pH 7.0) with 0.2 mM DTT, 1 mM $MgCl_2$, plus the following protease inhibitors:

| | |
|---|---|
| 0.7 μg/mL | Pepstatin A |
| 0.5 μg/mL | Leupeptin |
| 0.2 mM | phenylmethylsulfonyl fluoride (PMSF), and |
| 0.5 mM | EDTA, |

lysed by pretreatment with 200 μg/mL lysozyme and 3 μg/mL DNAase followed by sonication in 250 mL batches for 5 x 1.5 minutes on ice using a Branson Model 450 ultrasonic cell disrupter (Branson Sonic Power Co., Danbury CT). The *E. coli* cell lysates are then cleared by centrifugation at 35,000 X g for one hour followed by filtration through 0.45 micron filters. GP in the soluble fraction of the lysates (estimated to be less than 1% of the total protein) is purified by monitoring the enzyme activity (as described in GPa Activity Assay section, below) from a series of chromatographic steps detailed below.

Immobilized Metal Affinity Chromatography (IMAC)

This step is based on the method of Luong et al. (Luong et al. Journal of Chromatography (1992) 584, 77-84). 500 mL of the filtered soluble fraction of cell lysates (prepared from approximately 160 - 250 g of original cell pellet) are loaded onto a 130 mL column of IMAC Chelating-Sepharose (Pharmacia LKB Biotechnology, Piscataway, New Jersey) which has been charged with 50 mM $CuCl_2$ and 25 mM β-glycerophosphate, 250 mM NaCl and 1 mM imidazole at pH 7 equilibration buffer. The column is washed with equilibration buffer until the $A_{280}$ returns to baseline. The sample is then eluted from the column with the same buffer containing 100 mM imidazole to remove the bound GP and other bound proteins. Fractions containing the GP activity are pooled (approximately 600 mL), and ethylenediaminetetraacetic acid (EDTA), DL-dithiothreitol (DTT), phenylmethylsulfonyl fluoride (PMSF), leupeptin and pepstatin A are added to obtain 0.3 mM, 0.2 mM, 0.2 mM, 0.5 μg/mL and 0.7 μg/mL concentrations respectively. The pooled GP is desalted over a Sephadex G-25 column (Sigma Chemical Co., St. Louis, Missouri) equilibrated with 25 mM Tris-HCl (pH 7.3), 3 mM DTT buffer (Buffer A) to remove imidazole and is stored on ice until the second chromatographic step.

5'- AMP-Sepharose Chromatography

The desalted pooled GP sample (approximately 600mL) is next mixed with 70 mL of 5'-AMP Sepharose (Pharmacia LKB Biotechnology, Piscataway, New Jersey) which has been equilibrated with Buffer A (see above). The mixture is gently agitated for one hour at 22°C then packed into a column and washed with Buffer A until the $A_{280}$ returns to baseline. GP and other proteins are eluted from the column with 25 mM Tris-HCl, 0.2 mM DTT and 10 mM adenosine 5'-monophosphate (AMP) at pH 7.3 (Buffer B). GP-containing fractions are pooled following identification by determining enzyme (described below) activity and visualizing the $M_r$ approximately 97 kdal GP protein band by sodium dodecyl sulfate polyacrylamide gel electrophoresis (SDS-PAGE) followed by silver staining (2D-silver Stain II "Daiichi Kit", Daiichi Pure Chemicals Co., LTD., Tokyo, Japan) and then pooled. The pooled GP is dialyzed into 25 mM β-glycerophosphate, 0.2 mM DTT, 0.3 mM EDTA, 200 mM NaCl, pH 7.0 buffer (Buffer C) and stored on ice until use.

Prior to use of the GP enzyme, the enzyme is converted from the inactive form as expressed in *E. coli* strain XL-1 Blue (designated GPb) (Stratagene Cloning Systems, La Jolla, California), to the active form (designated GPa) by the procedure described in the section Activation of GP below.

Purification of Glycogen Phosphorylase expressed in Sf9 cells

The Sf9 cells in pellets described above are resuspended in 25 mM β-glycerophosphate (pH 7.0) with 0.2 mM DTT, 1 mM MgCl2, plus the following protease inhibitors:

| | |
|---|---|
| 0.7 μg/mL | Pepstatin A |

(continued)

| | |
|---|---|
| 0.5 μg/mL | Leupeptin |
| 0.2 mM | phenylmethylsulfonyl fluoride (PMSF), and |
| 0.5 mM | EDTA, |

then lysed by pretreatment with 3 μg/mL DNAase followed by sonication in batches for 3 x 1 minutes on ice using a Branson Model 450 ultrasonic cell disrupter (Branson Sonic Power Co., Danbury CT). The Sf9 cell lysates are then cleared by centrifugation at 35,000 X g for one hour followed by filtration through 0.45 micron filters. GP in the soluble fraction of the lysates (estimated to be 1.5% of the total protein) is purified by monitoring the enzyme activity (as described in GPa Activity Assay section, below) from a series of chromatographic steps detailed below

Immobilized Metal Affinity Chromatography (IMAC)

Immobilized Metal Affinity Chromatography is performed as described in the section above. The pooled, desalted GP is then stored on ice until further processed.

Activation of GP

Before further chromatography, the fraction of inactive enzyme as expressed in Sf9 cells (designated GPb) is converted to the active form (designated GPa) by the following procedure described in Activation of GP below.

Anion Exchange Chromatography Following activation of the IMAC purified GPb to GPa by reaction with the immobilized phosphorylase kinase, the pooled GPa fractions are dialyzed against 25 mM Tris-HCl, pH 7.5, containing 0.5 mM DTT, 0.2 mM EDTA, 1.0 mM phenylmethylsulfonyl fluoride (PMSF), 1.0 μg/mL leupeptin and 1.0 μg/mL pepstatin A. The sample is then loaded onto a MonoQ Anion Exchange Chromatography column (Pharmacia Biotech. Inc., Piscataway, New Jersey). The column is washed with equilibration buffer until the $A_{280}$ returns to baseline. The sample is then eluted from the column with a linear gradient of 0-0.25 M NaCl to remove the bound GP and other bound proteins. GP-containing fractions elute between 0.1-0.2 M NaCl range, as detected by monitoring the eluant for peak protein absorbance at $A_{280}$. The GP protein is then identified by visualizing the $M_r$ approximately 97 kdal GP protein band by sodium dodecyl sulfate polyacrylamide gel electrophoresis (SDS-PAGE) followed by silver staining (2D-silver Stain II "Daiichi Kit", Daiichi Pure Chemicals Co., Ltd., Tokyo, Japan) and then pooled. The pooled GP is dialyzed into 25 mM BES, 1.0 mM DTT, 0.5 mM EDTA, 5 mM NaCl, pH 6.8 buffer and stored on ice until use.

Determination of GP Enzyme Activity

Activation of GP: Conversion of GPb to GPa

Prior to the determination of GP enzyme activity, the enzyme is converted from the inactive form as expressed in *E. coli* strain XL-1 Blue (designated GPb) (Stratagene Cloning Systems, La Jolla, California), to the active form (designated GPa) by phosphorylation of GP using phosphorylase kinase as follows. The fraction of inactive enzyme as expressed in Sf9 cells (designated GPb) is also converted to the active form (designated GPa) by the follow procedure.

GP reaction with Immobilized Phosphorylase Kinase

Phosphorylase kinase (Sigma Chemical Company, St. Louis, MO) is immobilized on Affi-Gel 10 (BioRad Corp., Melville, NY) as per the manufacturer's instructions. In brief, the phosphorylase kinase enzyme (10 mg) is incubated with washed Affi-Gel beads (1 mL) in 2.5 mL of 100 mM HEPES and 80 mM $CaCl_2$ at pH 7.4 for 4 hours at 4°C. The Affi-Gel beads are then washed once with the same buffer prior to blocking with 50 mM HEPES and 1 M glycine methyl ester at pH 8.0 for one hour at room temperature. Blocking buffer is removed and replaced with 50 mM HEPES (pH 7.4), 1 mM β-mercaptoethanol and 0.2% $NaN_3$ for storage. Prior to use to convert GPb to GPa, the Affi-Gel immobilized phosphorylase kinase beads are equilibrated by washing in the buffer used to perform the kinase reaction, consisting of 25 mM β-glycerophosphate, 0.3 mM DTT, and 0.3mM EDTA at pH 7.8 (kinase assay buffer).

The partially purified, inactive GPb obtained from 5'-AMP-Sepharose chromatography above (from *E. coli*) or the mixture of GPa and GPb obtained from IMAC above (from Sf9 cells) is diluted 1:10 with the kinase assay buffer then mixed with the aforementioned phosphorylase kinase enzyme immobilized on the Affi-Gel beads. NaATP is added to 5 mM and $MgCl_2$ to 6 mM. The resulting mixture is mixed gently at 25°C for 30 to 60 minutes. The sample is removed

from the beads and the percent activation of GPb by conversion to GPa is estimated by determining GP enzyme activity in the presence and absence of 3.3 mM AMP. The percent of total GP enzyme activity due to GPa enzyme activity (AMP-independent) is then calculated as follows:

$$((\text{HLGP activity - AMP})/(\text{HLGP activity + AMP}))\ 100$$

Alternately, the conversion of GPb to GPa can be monitored by isoelectric focusing based on the shift in electrophoretic mobility that is noted following conversion of GPb to GPa. GP samples are analyzed by isoelectric focusing (IEF) utilizing the Pharmacia PfastGel System (Pharmacia Biotech. Inc., Piscataway, New Jersey) using precast gels (pl range 4-6.5) and the manufacturer's recommended method. The resolved GPa and GPb bands are then visualized on the gels by silver staining (2D-silver Stain II "Daiichi Kit", Daiichi Pure Chemicals Co., LTD., Tokyo, Japan). Identification of GPa and GPb is made by comparison to *E. coli* derived GPa and GPb standards that are run in parallel on the same gels as the experimental samples.

GPa Activity Assay

The effect of the compounds of Formula I or IA on the activity of the activated form of glycogen phosphorylase (GPa) can be determined by one of two methods; glycogen phosphorylase a activity is measured in the forward direction by monitoring the production of glucose-1-phosphate from glycogen or by following the reverse reaction, measuring glycogen synthesis from glucose-1-phosphate by the release of inorganic phosphate. All reactions are run in triplicate in 96-well microtiter plates and the change in absorbance due to formation of the reaction product is measured at the wavelength specified below in a MCC/340 MKII Elisa Reader (Lab Systems, Finland), connected to a Titertech Microplate Stacker (ICN Biomedical Co, Huntsville, Alabama).

To measure the GPa enzyme activity in the forward direction, the production of glucose-1-phosphate from glycogen is monitored by the multienzyme coupled general method of Pesce et al. (Pesce, M.A., Bodourian, S.H., Harris, R.C. and Nicholson, J.F. (1977) Clinical Chemistry 23, 1711-1717) modified as follows: 1 to 100 µg GPa, 10 units phosphoglucomutase and 15 units glucose-6-phosphate dehydrogenase (Boehringer Mannheim Biochemicals, Indianapolis, IN) are diluted to 1 mL in Buffer A (described hereinafter). Buffer A is at pH 7.2 and contains 50 mM HEPES, 100 mM KCl, 2.5 mM ethyleneglycoltetraacetic acid (EGTA), 2.5 mM $MgCl_2$, 3.5 mM $KH_2PO_4$ and 0.5 mM dithiothreitol. 20 µl of this stock is added to 80 µl of Buffer A containing 0.47 mg/mL glycogen, 9.4 mM glucose, 0.63 mM of the oxidized form of nicotinamide adenine dinucleotide phosphate (NADP+). The compounds to be tested are added as 5 µL of solution in 14% dimethylsulfoxide (DMSO) prior to the addition of the enzymes. The basal rate of GPa enzyme activity in the absence of inhibitors is determined by adding 5 µL of 14% DMSO and a fully-inhibited rate of GPa enzyme activity is obtained by adding 20 µL of 50 mM of the positive control test substance, caffeine. The reaction is followed at room temperature by measuring the conversion of oxidized NADP+ to reduced NADPH at 340 nm.

To measure the GPa enzyme activity in the reverse direction, the conversion of glucose-1-phosphate into glycogen plus inorganic phosphate is measured by the general method described by Engers et al. (Engers, H.D., Shechosky, S. and Madsen, N.B. (1970) Can. J. Biochem. 48, 746-754) modified as follows: 1 to 100 ug GPa is diluted to 1 mL in Buffer B (described hereinafter). Buffer B is at pH 7.2 and contains 50 mM HEPES, 100 mM KCl, 2.5 mM EGTA, 2.5 mM $MgCl_2$ and 0.5 mM dithiothreitol. 20 µL of this stock is added to 80 µL of Buffer B with 1.25 mg/mL glycogen, 9.4 mM glucose, and 0.63 mM glucose-1-phosphate. The compounds to be tested are added as 5 µL of solution in 14% DMSO prior to the addition of the enzyme. The basal rate of GPa enzyme activity in the absence of added inhibitors is determined by adding 5 µL of 14% DMSO and a fully-inhibited rate of GPa enzyme activity is obtained by adding 20 µL of 50 mM caffeine. This mixture is incubated at room temperature for 1 hour and the inorganic phosphate released from the glucose-1-phosphate is measured by the general method of Lanzetta et al. (Lanzetta, P.A., Alvarez, L.J., Reinach, P.S. and Candia, O.A. (1979) Anal. Biochem. 100, 95-97) modified as follows: 150 µL of 10 mg/mL ammonium molybdate, 0.38 mg/mL malachite green in 1 N HCl is added to 100 µL of the enzyme mix. After a 20 minute incubation at room temperature, the absorbance is measured at 620 nm.

The above assays can also be used to assess activity of glycogen phosphorylase derived from various pathogenic sources. Adaptation of the assays as required is easily accomplished.

The above assays carried out with a range of concentrations of test compound allows the determination of an $IC_{50}$ value (as defined hereinabove, see e.g., p. 14) for the *in vitro* inhibition of GPa enzyme activity by that test compound.

The inhibiting effect of compounds employed in the invention on the human liver and human muscle glycogen phosphorylase a isoforms is shown in Table 1 below.

TABLE 1*

| Compound Name | HLGPa $IC_{50}$ nM | HMGPa $IC_{50}$ nM |
|---|---|---|
| 5-chloro-1H-indole-2-carboxylic acid [(1S)-benzyl-(2R)-hydroxy-3-((3S)-hydroxy-pyrrolidin-1-yl)-3-oxopropyl]-amide | 54 | 96 |
| 5-chloro-1H-indole-2-carboxylic acid [(1S)-benzyl-(2R)-hydroxy-3-((3S, 4S)-dihydroxy-pyrrolidin-1-yl)-3-oxopropyl]-amide | 73 | 90 |
| 5-chloro-1H-indole-2-carboxylic acid [(1S)-benzyl-3-((3-hydroxy azetidin-1-yl)-(2R)-hydroxy-3-oxopropyl]-amide | 236 | 706 |
| 5-chloro-1H-indole-2-carboxylic acid [(1S)-benzyl-3-(cis-3,4-dihydroxy-pyrrolidin-1-yl)-(2R)-hydroxy-3-oxopropyl]-amide | 59 | 385 |
| 5-chloro-1H-indole-2-carboxylic acid [1-benzyl-2-(3-hydroxypyrrolidin-1-yl)-2-oxo-ethyl]-amide | 45 | 85 |
| 5-chloro-1H-indole-2-carboxylic acid [(1S)-benzyl-2-(cis-3,4-dihydroxypyrrolidin-1-yl)-2-oxo-ethyl]-amide | 30 | 97 |
| 5-chloro-1H-indole-2-carboxylic acid [(1S)-(4-fluorobenzyl-2-(4-hydroxy-piperidin-1-yl)-2-oxo-ethyl]-amide | 142 | 83 |
| 5-chloro-1H-indole-2-carboxylic acid (2-oxo-2-thiazolidin-3-yl-ethyl)-amide | 307 | 433 |
| 5-chloro-1H-indole-2-carboxylic acid [(1S)-benzyl-2-(3-hydroxy-azetidin-1-yl)-2-oxo-ethyl]-amide | 65 | 121 |
| 5-chloro-1H-indole-2-carboxylic acid [(1S)-benzyl-2-(3-hydroxyimino-azetidin-1-yl)-2-oxo-ethyl]-amide | 65 | 84 |
| 5-chloro-1 H-indole-2-carboxylic acid [(1S)-benzyl-2-((3S,4S)-dihydroxy-pyrrolidin-1-yl)-2-oxo-ethyl]-amide | 137 | 71 |
| *data are for HLGPa and HMGPa enzyme activity ($IC_{50}$) as determined by the reverse direction assay. | | |

Generally, the glycogen phosphorylase inhibitors are administered orally, but parenteral administration (e.g., intravenous, intramuscular, subcutaneous or intramedullary) may be utilized, for example, where oral administration is inappropriate or where the patient is unable to ingest the drug. For certain tissues such as the eye, topical administration may also be suitable.

The glycogen phosphorylase inhibitors may be administered alone or in combination with pharmaceutically acceptable carriers, in either single or multiple doses. Suitable pharmaceutical carriers include inert solid diluents or fillers, sterile aqueous solutions, oils (e.g., peanut oil, sesame oil) and various organic solvents. The pharmaceutical compositions formed by combining the active compounds and pharmaceutically acceptable carriers can then be readily administered in a variety of dosage forms such as tablets, powders, lozenges, emulsions, oil soft gels, syrups, injectable solutions, spray-dried formulations, transdermal or transmucosal patches, inhalable formulations and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, methylcellulose, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions containing an active compound or a pharmaceutically acceptable salt thereof in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. The sterile aqueous media employed are all readily available by standard techniques known to those skilled in the art.

Methods of preparing various pharmaceutical compositions with a certain amount of active ingredient are known, or will be apparent in light of this disclosure, to those skilled in this art. For examples of how to prepare such compositions see Remington's Pharmaceutical Sciences, Mack Publishing Company, Easton, Pa., 19th Edition (1995). Pharmaceutical compositions administered according to the invention generally contain 0.01%-95% of glycogen phosphorylase inhibitor, preferably 1%-70%. In any event, the composition or formulation to be administered contains a quantity of a glycogen phosphorylase inhibitor in an amount effective to inhibit tumor growth. Typically, an effective dosage for the glycogen phosphorylase inhibitor is in the range of about 0.005 to 50 mg/kg/day, preferably 0.01 to 25 mg/kg/day and most preferably 0.1 to 15 mg/kg/day.

## Claims

1. A method of inhibiting tumor growth in a mammal, comprising administering to the mammal an effective amount of a compound which is a glycogen phosphorylase inhibitor of Formula I or Formula IA

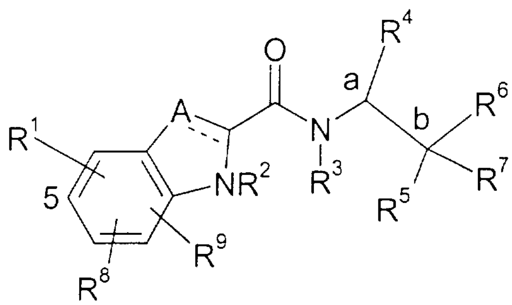

**Formula I**

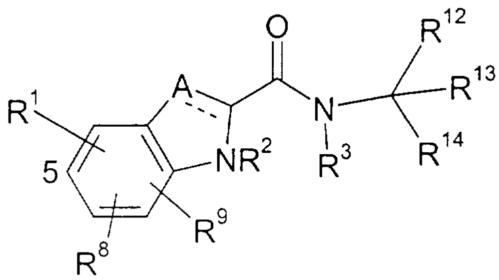

**Formula IA**

and the pharmaceutically acceptable salts and prodrugs thereof, wherein

the dotted line (---) is an optional bond;
A is —CH=, —C(($C_1$-$C_4$)alkyl)= or —C(halo)= when the dotted line (---) is a bond, or A is methylene or —CH (($C_1$-$C_4$)alkyl)— when the dotted line (---) is not a bond;
$R^1$, $R^8$ and $R^9$ are each independently H, halo, 4-, 6- or 7-nitro, cyano, ($C_1$-$C_4$)alkyl, ($C_1$-$C_4$)alkoxy, fluoromethyl, difluoromethyl or trifluoromethyl;
$R^2$ is H;
$R^3$ is H or ($C_1$-$C_5$)alkyl;
$R^4$ is H, methyl, ethyl, n-propyl, hydroxy($C_1$-$C_3$)alkyl, ($C_1$-$C_3$)alkoxy($C_1$-$C_3$)alkyl, phenyl($C_1$-$C_4$)alkyl, phenylhydroxy($C_1$-$C_4$)alkyl, phenyl($C_1$-$C_4$)alkoxy($C_1$-$C_4$)alkyl, thien-2- or-3-yl($C_1$-$C_4$)alkyl or fur-2- or -3-yl($C_1$-$C_4$) alkyl wherein said $R^4$ rings are mono-, di- or tri-substituted independently on carbon with H, halo, ($C_1$-$C_4$)alkyl, ($C_1$-$C_4$)alkoxy, trifluoromethyl, hydroxy, amino or cyano; or
$R^4$ is pyrid-2-, -3- or -4-yl($C_1$-$C_4$)alkyl, thiazol-2-, -4- or -5-yl($C_1$-$C_4$)alkyl, imidazol -1-,-2-, -4- or -5-yl($C_1$-$C_4$) alkyl, pyrrol-2- or -3-yl($C_1$-$C_4$)alkyl, oxazol-2-, -4- or -5-yl-($C_1$-$C_4$)alkyl, pyrazol-3-, -4- or -5-yl($C_1$-$C_4$)alkyl, iso-

xazol-3-, -4- or -5-yl($C_1$-$C_4$)alkyl, isothiazol-3-, -4- or -5-yl($C_1$-$C_4$)alkyl, pyridazin-3- or -4-yl-($C_1$-$C_4$)alkyl, pyrimidin-2-, -4-, -5- or -6-yl($C_1$-$C_4$)alkyl, pyrazin-2- or -3-yl($C_1$-$C_4$)alkyl or 1,3,5-triazin-2-yl($C_1$-$C_4$)alkyl, wherein said preceding $R^4$ heterocycles are optionally mono- or di-substituted independently with halo, trifluoromethyl, ($C_1$-$C_4$)alkyl, ($C_1$-$C_4$)alkoxy, amino or hydroxy and said mono-or di-substituents are bonded to carbon;

$R^5$ is H, hydroxy, fluoro, ($C_1$-$C_5$)alkyl, ($C_1$-$C_5$)alkoxy, ($C_1$-$C_6$)alkanoyl, amino($C_1$-$C_4$)alkoxy, mono-N- or di-N, N-($C_1$-$C_4$)alkylamino($C_1$-$C_4$)alkoxy, carboxy($C_1$-$C_4$)alkoxy, ($C_1$-$C_5$)alkoxy-carbonyl($C_1$-$C_4$)alkoxy, benzyloxy-carbonyl($C_1$-$C_4$)alkoxy, or carbonyloxy wherein said carbonyloxy is carbon-carbon linked with phenyl, thiazolyl, imidazolyl, 1H-indolyl furyl, pyrrolyl, oxazolyl, pyrazolyl, isoxazolyl, isothiazolyl, pyridazinyl, pyrimidinyl, pyrazinyl or 1,3,5-triazinyl and wherein said preceding $R^5$ rings are optionally mono-substituted with halo, ($C_1$-$C_4$) alkyl, ($C_1$-$C_4$)alkoxy, hydroxy, amino or trifluoromethyl and said mono-substituents are bonded to carbon;

$R^7$ is H, fluoro or ($C_1$-$C_5$)alkyl; or

$R^5$ and $R^7$ can be taken together to be oxo;

$R^6$ is C(O)$R^{10}$, C(O)N$R^{16}R^{17}$, ($C_1$-$C_8$)alkoxycarbonyl or carboxy,

wherein $R^{16}$ is H, hydroxy, or ($C_1$-$C_3$ alkoxy), wherein $R^{17}$ is H, ($C_1$-$C_8$)alkyl, hydroxy, ($C_1$-$C_8$)alkoxy, methylene-perfluorinated($C_1$-$C_8$) alkyl, phenyl, pyridyl, thienyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, pyridazinyl, pyrimidinyl, pyrazinyl, piperazinyl or 1,3,5-triazinyl wherein said preceding $R^{17}$ rings are carbon-nitrogen linked; or $R^{17}$ is mono-, di-or tri-substituted ($C_1$-$C_5$)alkyl, wherein said substituents are independently H, hydroxy, amino, mono-N- or di-N,N-($C_1$-$C_5$)alkylamino; or $R^{17}$ is mono- or di-substituted ($C_1$-$C_5$ alkyl), wherein said substituents are independently phenyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazolinyl, piperazinyl or 1,3,5-triazolinyl, wherein the nonaromatic nitrogen-containing $R^{17}$ rings are optionally mono-substituted on nitrogen with ($C_1$-$C_6$)alkyl, benzyl, benzoyl or ($C_1$-$C_6$)alkoxycarbonyl and wherein the $R^{17}$ rings are optionally mono-substituted on carbon with halo, ($C_1$-$C_4$)alkyl, ($C_1$-$C_4$) alkoxy, hydroxy, amino, or mono-N- or di-N,N($C_1$-$C_5$)alkylamino provided that no quaternized nitrogen is included and there are no nitrogen-oxygen, nitrogen-nitrogen or nitrogen-halo bonds;

$R^{10}$ is piperazin-1-yl, 4-($C_1$-$C_4$)alkylpiperazin-1-yl, 4-formylpiperazin-1-yl, morpholino, thiomorpholino, 1-oxothiomorpholino, 1,1-dioxo-thiomorpholino, thiazolidin-3-yl, 1-oxo-thiazolidin-3-yl, 1,1-dioxo-thiazolidin-3-yl, 2-($C_1$-$C_6$)alkoxycarbonylpyrrolidin-1-yl, oxazolidin-3-yl or 2(R)-hydroxymethylpyrrolidin-1-yl; or

$R^{10}$ is 3- and/or 4-mono-or di-substituted oxazetidin-2-yl, 2-, 4-, and/or 5- mono- or di-substituted oxazolidin-3-yl, 2-, 4-, and/or 5- mono- or di- substituted thiazolidin-3-yl, 2-, 4-, and/or 5- mono- or di- substituted 1-oxothiazolidin-3-yl, 2-, 4-, and/or 5- mono- or di-substituted 1,1-dioxothiazolidin-3-yl, 3- and/or 4-, mono- or di-substituted pyrrolidin-1-yl, 3-, 4-and/or 5-, mono-, di- or tri-substituted piperidin-1-yl, 3-, 4-, and/or 5- mono-, di-, or tri-substituted piperazin-1-yl, 3-substituted azetidin-1-yl, 4- and/or 5-, mono- or di-substituted 1,2-oxazinan-2-yl, 3-and/or 4-mono- or di-substituted pyrazolidin-1-yl, 4- and/or 5-, mono- or di-substituted isoxazolidin-2-yl, 4- and/or 5-, mono- and/or di-substituted isothiazolidin-2-yl wherein said $R^{10}$ substituents are independently H, halo, ($C_1$-$C_5$)-alkyl, hydroxy, amino, mono-N- or di-N,N-($C_1$-$C_5$)alkylamino, formyl, oxo, hydroxyimino, ($C_1$-$C_5$)alkoxy, carboxy, carbamoyl, mono-N-or di-N,N-($C_1$-$C_4$)alkylcarbamoyl, ($C_1$-$C_4$)alkoxyimino, ($C_1$-$C_4$)alkoxymethoxy, ($C_1$-$C_6$)alkoxycarbonyl, carboxy($C_1$-$C_5$)alkyl or hydroxy($C_1$-$C_5$)alkyl;

$R^{12}$ is H, methyl, ethyl, n-propyl, hydroxy($C_1$-$C_3$)alkyl, ($C_1$-$C_3$)alkoxy($C_1$-$C_3$)alkyl, phenyl($C_1$-$C_4$)alkyl, phenylhydroxy($C_1$-$C_4$)alkyl, (phenyl)(($C_1$-$C_4$)-alkoxy)($C_1$-$C_4$)alkyl, thien-2-or -3-yl($C_1$-$C_4$)alkyl or fur-2- or -3-yl($C_1$-$C_4$) alkyl wherein said $R^{12}$ rings are mono-, di- or tri-substituted independently on carbon with H, halo, ($C_1$-$C_4$) alkyl, ($C_1$-$C_4$)alkoxy, trifluoromethyl, hydroxy, amino, cyano or 4,5-dihydro-1H-imidazol-2-yl; or

$R^{12}$ is pyrid-2-, -3- or -4-yl($C_1$-$C_4$)alkyl, thiazol-2-, -4- or -5-yl($C_1$-$C_4$)alkyl, imidazol-2-,-4- or -5-yl($C_1$-$C_4$)alkyl, pyrrol-2- or -3-yl($C_1$-$C_4$)alkyl, oxazol-2-, -4- or -5-yl($C_1$-$C_4$)alkyl, pyrazol-3-, -4- or -5-yl($C_1$-$C_4$)alkyl, isoxazol-3-, -4- or -5-yl($C_1$-$C_4$)alkyl, isothiazol-3-, -4- or -5-yl($C_1$-$C_4$)alkyl, pyridazin-3- or -4-yl($C_1$-$C_4$)alkyl, pyrimidin-2-, -4-, -5- or -6-yl($C_1$-$C_4$)alkyl, pyrazin-2-or -3-yl($C_1$-$C_4$)alkyl, 1,3,5-triazin-2-yl($C_1$-$C_4$)alkyl or indol-2-($C_1$-$C_4$) alkyl, wherein said preceding $R^{12}$ heterocycles are optionally mono- or di-substituted independently with halo, trifluoromethyl, ($C_1$-$C_4$)alkyl, ($C_1$-$C_4$)alkoxy, amino, hydroxy or cyano and said substituents are bonded to carbon; or

$R^{12}$ is $R^{11}$-carbonyloxymethyl, wherein said $R^{11}$ is phenyl, thiazolyl, imidazolyl, 1H-indolyl, furyl, pyrrolyl, oxazolyl, pyrazolyl, isoxazolyl, isothiazolyl, pyridyl, pyridazinyl, pyrimidinyl, pyrazinyl or 1,3,5-triazinyl and wherein said preceding $R^{11}$ rings are optionally mono- or di-substituted independently with halo, amino, hydroxy, ($C_1$-$C_4$)alkyl, ($C_1$-$C_4$)alkoxy or trifluoromethyl and said mono- or di-substituents are bonded to carbon;

$R^{13}$ is H, methyl, ethyl, n-propyl, hydroxymethyl, or hydroxyethyl;

$R^{14}$ is C(O)$R^{15}$ carboxy, ($C_1$-$C_8$)alkoxycarbonyl, benzyloxycarbonyl, or CON$R^{18}R^{19}$;

$R^{15}$ is morpholino, thiomorpholino, 1-oxothiomorpholino, 1,1-dioxothiomorpholino, thiazolidin-3-yl, 1-oxothiazolidin-3-yl, 1,1-dioxothiazolidin-3-yl, pyrrolidin-1-yl, piperidin-1-yl, piperazin-1-yl, piperazin-4-yl, azetidin-1-yl,

1,2-oxazinan-2-yl, pyrazolidin-1-yl, isoxazolidin-2-yl, isothiazolidin-2-yl, 1,2-oxazetidin-2-yl, oxazolidin-3-yl, 3,4-dihydroisoquinolin-2-yl, 1,3-dihydroisoindol-2-yl, 3,4-dihydro-2H-quinol-1-yl, 2,3-dihydro-benzo[1,4]oxazin-4-yl, 2,3-dihydro-benzo[1,4]-thiazine-4-yl, 3,4-dihydro-2H-quinoxalin-1-yl, 3,4-dihydro-benzo[c][1,2]oxazin-1-yl, 1,4-dihydro-benzo[d][1,2]oxazin-3-yl, 3,4-dihydro-benzo[e][1,2]-oxazin-2-yl, 3H-benzo[d]isoxazol-2-yl, 3H-benzo[c]isoxazol-1-yl or azepan-1-yl,

wherein said $R^{15}$ rings are optionally mono-, di- or tri-substituted independently with halo, $(C_1-C_5)$alkyl, $(C_1-C_5)$alkoxy, hydroxy, amino, mono-N- or di-N,N-$(C_1-C_5)$alkylamino, formyl, carboxy, carbamoyl, mono-N- or di-N,N-$(C_1-C_5)$alkylcarbamoyl, $(C_1-C_6)$alkoxy$(C_1-C_3)$alkoxy, $(C_1-C_5)$alkoxycarbonyl, benzyloxycarbonyl, $(C_1-C_5)$alkoxycarbonyl$(C_1-C_5)$alkyl, $(C_1-C_4)$alkoxycarbonylamino, carboxy$(C_1-C_5)$alkyl, carbamoyl$(C_1-C_5)$alkyl, mono-N- or di-N,N-$(C_1-C_5)$alkylcarbamoyl$(C_1-C_5)$alkyl, hydroxy$(C_1-C_5)$alkyl, $(C_1-C_4)$alkoxy$(C_1-C_4)$alkyl, amino $(C_1-C_4)$alkyl, mono-N- or di-N,N-$(C_1-C_4)$alkylamino$(C_1-C_4)$alkyl, oxo, hydroxyimino or $(C_1-C_6)$alkoxyimino and wherein no more than two substituents are selected from oxo, hydroxyimino or $(C_1-C_6)$alkoxyimino and oxo, hydroxyimino or $(C_1-C_6)$alkoxyimino are on nonaromatic carbon; and
wherein said $R^{15}$ rings are optionally additionally mono- or di-substituted independently with $(C_1-C_5)$alkyl or halo;
wherein $R^{18}$ is H, $(C_1-C_6)$alkyl, cyclo$(C_3-C_6)$alkyl, cyclo$(C_3-C_6)$alkyl$(C_1-C_5)$alkyl, hydroxy or $(C_1-C_8)$alkoxy; and wherein $R^{19}$ is H, cyclo$(C_3-C_8)$alkyl, cyclo$(C_3-C_8)$alkyl$(C_1-C_5)$alkyl, cyclo$(C_4-C_7)$alkenyl, cyclo$(C_3-C_7)$alkyl $(C_1-C_5)$alkoxy, cyclo$(C_3-C_7)$alkoxy, hydroxy, methylene-perfluorinated$(C_1-C_8)$alkyl, phenyl, or a heterocycle wherein said heterocycle is pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl, 1,3,5-triazinyl, benzothiazolyl, benzoxazolyl, benzimidazolyl, thiochromanyl or tetrahydrobenzothiazolyl wherein said heterocycle rings are carbon-nitrogen linked; or $R^{19}$ is $(C_1-C_6)$alkyl or $(C_1-C_8)$alkoxy wherein said $(C_1-C_8)$alkoxy is optionally monosubstituted with cyclo$(C_4-C_7)$alken-1-yl, phenyl, thienyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, thiomorpholinyl, 1-oxo-thiomorpholinyl, 1,1-dioxothiomorpholinyl, pyridazinyl, pyrimidinyl, pyrazolinyl, piperazinyl, 1,3,5-triazinyl or indolyl and wherein said $(C_1-C_6)$alkyl or $(C_1-C_8)$alkoxy are optionally additionally independently mono- or di-substituted with halo, hydroxy, $(C_1-C_5)$alkoxy, amino, mono-N- or Di-N,N-$(C_1-C_5)$alkylamino, cyano, carboxy, or $(C_1-C_4)$alkoxycarbonyl, and wherein the $R^{19}$ rings are optionally mono- or di-substituted independently on carbon with halo, $(C_1-C_4)$alkyl, $(C_1-C_4)$alkoxy, hydroxy, hydroxy$(C_1-C_4)$alkyl, amino$(C_1-C_4)$alkyl, mono-N- or di-N,N-$(C_1-C_4)$alkylamino, cyano, carboxy, $(C_1-C_5)$alkoxycarbonyl, carbamoyl, formyl or trifluoromethyl and said $R^{19}$ rings may optionally be additionally mono- or di-substituted independently with $(C_1-C_5)$alkyl or halo, with the proviso that no quaternized nitrogen on any $R^{19}$ heterocycle is included, with the further provisos that when $R^{12}$ is benzyl and $R^{13}$ is methyl, $R^{15}$ is not 4-hydroxy-piperidin-1-yl, and when $R^{12}$ is benzyl and $R^{13}$ is methyl, $R^{15}$ is not $C(O)N(CH_3)_2$.

**2.** The method of claim 1, wherein the compound is of Formula I and wherein

$R^1$ is 5-H, 5-halo, 5-methyl, 5-cyano or 5-trifluoromethyl;
$R^8$ and $R^9$ are each independently H or halo;
A is —C(H)=;
$R^2$ and $R^3$ are H;
$R^4$ is phenyl$(C_1-C_2)$alkyl wherein said phenyl groups are mono-, di- or tri-substituted independently with H or halo or mono- or di- substituted independently with H, halo, $(C_1-C_4)$alkyl, $(C_1-C_4)$alkoxy, trifluoromethyl, hydroxy, amino or cyano; or
$R^4$ is thien-2- or -3-yl$(C_1-C_2)$alkyl, pyrid-2-, -3- or -4-yl$(C_1-C_2)$alkyl, thiazol-2-,-4- or -5-yl$(C_1-C_2)$alkyl, imidazol -1-, -2-, -4- or -5-yl$(C_1-C_2)$alkyl, fur-2- or -3-yl$(C_1-C_2)$alkyl, pyrrol-2- or -3-yl$(C_1-C_2)$alkyl, oxazol-2-, -4- or -5-yl-$(C_1-C_2)$alkyl, pyrazol-3-, -4- or -5-yl$(C_1-C_2)$alkyl, isoxazol-3-, -4- or -5-yl$(C_1-C_2)$alkyl wherein said preceding $R_4$ heterocycles are optionally mono- or di-substituted independently with halo, trifluoromethyl, $(C_1-C_4)$alkyl, $(C_1-C_4)$alkoxy, amino or hydroxy and said mono- or di-substituents are bonded to carbon;
$R^5$ is hydroxy;
$R^6$ is $C(O)R^{10}$; and
$R^7$ is H.

**3.** The method of claim 2, wherein

the carbon atom labelled (a) has (S) stereochemistry;

the carbon atom labelled (b) has (R) stereochemistry;
$R^4$ is phenyl($C_1$-$C_2$)alkyl, thien-2-yl-($C_1$-$C_2$)alkyl, thien-3-yl-($C_1$-$C_2$)alkyl, fur-2-yl-($C_1$-$C_2$)alkyl or fur-3-yl-($C_1$-$C_2$)alkyl wherein said rings are mono- or di- substituted independently with H or fluoro; and
$R^{10}$ is morpholino, 4-($C_1$-$C_4$)alkylpiperazin-1-yl, 3-substituted azetidin-1-yl, 3-and/or 4-, mono- or di-substituted pyrrolidin-1-yl, 4- and/or 5- mono- or di-substituted isoxazolidin-2-yl, 4- and/or 5-, mono- or di-substituted 1,2-oxazinan-2-yl wherein said substituents are each independently H, halo, hydroxy, amino, mono-N- or di-N, N-($C_1$-$C_6$)alkylamino, oxo, hydroxyimino or alkoxy.

**4.** The method of claim 3, wherein the compound is selected from among:

5-Chloro-1H-indole-2-carboxylic acid [(1S)-benzyl-(2R)-hydroxy-3-(4-methyl-piperazin-1-yl)-3-oxo-propyl]-amide hydrochloride,
5-Chloro-1H-indole-2-carboxylic acid [(1S)-benzyl-(2R)-hydroxy-3-(3-hydroxy-azetidin-1-yl)-3-oxo-propyl]-amide,
5-Chloro-1H-indole-2-carboxylic acid ((1S)-benzyl-(2R)-hydroxy-3-isoxazolidin-2-yl-3-oxo-propyl)-amide,
5-Chloro-1H-indole-2-carboxylic acid ((1S)-benzyl-(2R)-hydroxy-3-[1,2]oxazinan-2-yl-3-oxo-propyl)-amide,
5-Chloro-1H-indole-2-carboxylic acid [(1S)-benzyl-(2R)-hydroxy-3-((3S)-hydroxy-pyrrolidin-1-yl)-3-oxo-propyl]-amide,
5-Chloro-1H-indole-2-carboxylic acid [(1S)-benzyl-3-((3S,4S)-dihydroxy-pyrrolidin-1-yl)-(2R)-hydroxy-3-oxo-propyl]-amide,
5-Chloro-1H-indole-2-carboxylic acid [(1S)-benzyl-3-(cis-3,4-dihydroxy-pyrrolidin-1-yl)-(2R)-hydroxy-3-oxo-propyl]-amide, and
5-Chloro-1H-indole-2-carboxylic acid ((1S)-benzyl-(2R)-hydroxy-3-morpholin-4-yl-3-oxo-propyl)-amide.

**5.** The method of claim 3, wherein $R^1$ is 5-chloro, $R^8$ and $R^9$ are both H, $R^4$ is benzyl; and $R^{10}$ is selected from the group consisting of 4-methylpiperazin-1-yl, 3-hydroxyazetidin-1-yl, isoxazolidin-2-yl, (1,2)-oxazinan-2-yl, 3(S)-hydroxypyrrolidin-1-yl, (3S,4S)-dihydroxypyrrolidin-1-yl, cis-3,4-dihydroxypyrrolidin-1-yl; and morpholino.

**6.** The method of claim 1, wherein

$R^1$ is H, halo, methyl or cyano;
$R^8$ and $R^9$ are each independently H or halo;
A is —C(H)=;
$R^2$ and $R^3$ are H;
$R^4$ is phenyl($C_1$-$C_2$)alkyl wherein said phenyl groups are mono-, di- or tri-substituted independently with H or halo or mono- or di- substituted independently with H, halo, ($C_1$-$C_4$)alkyl, ($C_1$-$C_4$)alkoxy, trifluoromethyl, hydroxy, amino or cyano; or
$R^4$ is thien-2- or -3-yl($C_1$-$C_2$)alkyl, pyrid-2-, -3- or -4-yl($C_1$-$C_2$)alkyl, thiazol-2-, -4- or -5-yl($C_1$-$C_2$)alkyl, imidazol -1-, -2-, -4- or -5-yl($C_1$-$C_2$)alkyl, fur-2- or -3-yl($C_1$-$C_2$)alkyl, pyrrol-2- or-3-yl($C_1$-$C_2$)alkyl, oxazol-2-, -4- or -5-yl-($C_1$-$C_2$)alkyl, pyrazol-3-, -4- or -5-yl($C_1$-$C_2$)alkyl, isoxazol-3-, -4- or -5-yl($C_1$-$C_2$)alkyl wherein said preceding $R^4$ heterocycles are optionally mono- or di-substituted independently with halo, trifluoromethyl, ($C_1$-$C_4$) alkyl, ($C_1$-$C_4$)alkoxy, amino or hydroxy and said mono- or di-substituents are bonded to carbon;
$R^5$ is fluoro, ($C_1$-$C_4$)alkyl, ($C_1$-$C_5$)alkoxy, amino($C_1$-$C_4$)alkoxy, mono-N- or di-N,N-($C_1$-$C_4$)alkylamino($C_1$-$C_4$) alkoxy, carboxy($C_1$-$C_4$)alkoxy, ($C_1$-$C_5$)alkoxy-carbonyl($C_1$-$C_4$)alkoxy, benzyloxycarbonyl($C_1$-$C_4$)alkoxy;
$R^6$ is C(O)$R^{10}$; and
$R^7$ is H, fluoro or ($C_1$-$C_6$)alkyl.

**7.** The method of claim 1, wherein the tumor growth is dependent on glycogen phosphorylase activity.

**8.** The method of claim 1, wherein the tumor is a colorectal, lung or breast tumor, such as an adenocarcinoma.

**9.** A method of inhibiting tumor growth in a mammal comprising administering to said mammal an effective amount of a compound which is a glycogen phosphorylase inhibitor, wherein the inhibition of recombinant human liver glycogen phosphorylase by the compound, in the presence of 7.5 mM glucose, is **characterized by** an $IC_{50}$ having a value less than 100 nanomolar.

**10.** A pharmaceutical composition for the inhibition of tumor growth in a mammal comprising an effective amount of a glycogen phosphorylase inhibitor, or a pharmaceutically acceptable salt or prodrug thereof, in combination with a

pharmaceutically acceptable carrier, wherein the glycogen phosphorylase inhibitor is of Formula I or Formula IA

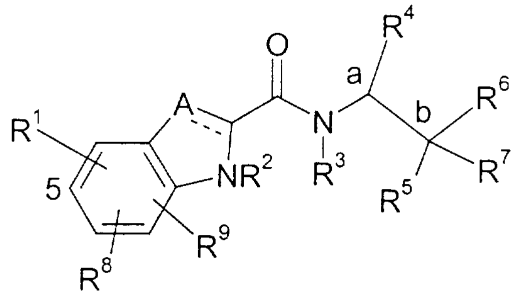

**Formula I**

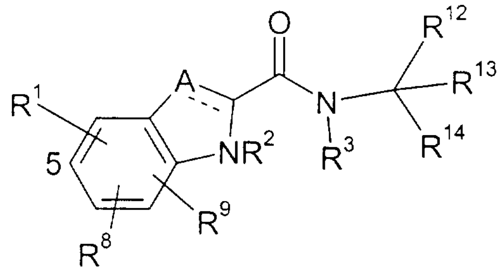

**Formula IA**

and the pharmaceutically acceptable salts and prodrugs thereof, wherein

the dotted line (---) is an optional bond;
A is —CH=, —C(($C_1$-$C_4$)alkyl)= or —C(halo)= when the dotted line (---) is a bond, or A is methylene or —CH (($C_1$-$C_4$)alkyl)- when the dotted line (---) is not a bond;
$R^1$, $R^8$ and $R^9$ are each independently H, halo, 4-, 6- or 7-nitro, cyano, ($C_1$-$C_4$)alkyl, ($C_1$-$C_4$)alkoxy, fluoromethyl, difluoromethyl or trifluoromethyl;
$R^2$ is H;
$R^3$ is H or ($C_1$-$C_5$)alkyl;
$R^4$ is H, methyl, ethyl, n-propyl, hydroxy($C_1$-$C_3$)alkyl, ($C_1$-$C_3$)alkoxy($C_1$-$C_3$)alkyl, phenyl($C_1$-$C_4$)alkyl, phenylhydroxy($C_1$-$C_4$)alkyl, phenyl($C_1$-$C_4$)alkoxy($C_1$-$C_4$)alkyl, thien-2- or-3-yl($C_1$-$C_4$)alkyl or fur-2- or -3-yl($C_1$-$C_4$) alkyl wherein said $R^4$ rings are mono-, di- or tri-substituted independently on carbon with H, halo, ($C_1$-$C_4$)alkyl, ($C_1$-$C_4$)alkoxy, trifluoromethyl, hydroxy, amino or cyano; or
$R^4$ is pyrid-2-, -3- or -4-yl($C_1$-$C_4$)alkyl, thiazol-2-, -4- or -5-yl($C_1$-$C_4$)alkyl, imidazol -1-,-2-, -4- or -5-yl($C_1$-$C_4$) alkyl, pyrrol-2- or -3-yl($C_1$-$C_4$)alkyl, oxazol-2-, -4- or -5-yl-($C_1$-$C_4$)alkyl, pyrazol-3-, -4- or -5-yl($C_1$-$C_4$)alkyl, isoxazol-3-, -4- or -5-yl($C_1$-$C_4$)alkyl, isothiazol-3-, -4- or -5-yl($C_1$-$C_4$)alkyl, pyridazin-3- or -4-yl-($C_1$-$C_4$)alkyl, pyrimidin-2-, -4-, -5- or -6-yl($C_1$-$C_4$)alkyl, pyrazin-2- or -3-yl($C_1$-$C_4$)alkyl or 1,3,5-triazin-2-yl($C_1$-$C_4$)alkyl, wherein said preceding $R^4$ heterocycles are optionally mono- or di-substituted independently with halo, trifluoromethyl, ($C_1$-$C_4$)alkyl, ($C_1$-$C_4$)alkoxy, amino or hydroxy and said mono-or di-substituents are bonded to carbon;
$R^5$ is H, hydroxy, fluoro, ($C_1$-$C_5$)alkyl, ($C_1$-$C_5$)alkoxy, ($C_1$-$C_6$)alkanoyl, amino($C_1$-$C_4$)alkoxy, mono-N- or di-N, N-($C_1$-$C_4$)alkylamino($C_1$-$C_4$)alkoxy, carboxy($C_1$-$C_4$)alkoxy, ($C_1$-$C_5$)alkoxy-carbonyl($C_1$-$C_4$)alkoxy, benzyloxycarbonyl($C_1$-$C_4$)alkoxy, or carbonyloxy wherein said carbonyloxy is carbon-carbon linked with phenyl, thiazolyl, imidazolyl, 1H-indolyl, furyl, pyrrolyl, oxazolyl, pyrazolyl, isoxazolyl, isothiazolyl, pyridazinyl, pyrimidinyl, pyrazinyl or 1,3,5-triazinyl and wherein said preceding $R^5$ rings are optionally mono-substituted with halo, ($C_1$-$C_4$)alkyl, ($C_1$-$C_4$)alkoxy, hydroxy, amino or trifluoromethyl and said mono-substituents are bonded to carbon;
$R^7$ is H, fluoro or ($C_1$-$C_5$)alkyl; or
$R^5$ and $R^7$ can be taken together to be oxo;

$R^6$ is $C(O)R^{10}$, $C(O)NR^{16}R^{17}$, $(C_1\text{-}C_8)$alkoxycarbonyl or carboxy,

wherein $R^{16}$ is H, hydroxy, or $(C_1\text{-}C_3$ alkoxy), wherein $R^{17}$ is H, $(C_1\text{-}C_8)$alkyl, hydroxy, $(C_1\text{-}C_8)$alkoxy, methylene-perfluorinated$(C_1\text{-}C_8)$ alkyl, phenyl, pyridyl, thienyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, pyridazinyl, pyrimidinyl, pyrazinyl, piperazinyl or 1,3,5-triazinyl wherein said preceding $R^{17}$ rings are carbon-nitrogen linked; or $R^{17}$ is mono-, di-or tri-substituted $(C_1\text{-}C_5)$alkyl, wherein said substituents are independently H, hydroxy, amino, mono-N- or di-N,N-$(C_1\text{-}C_5)$alkylamino; or $R^{17}$ is mono- or di-substituted $(C_1\text{-}C_5$ alkyl), wherein said substituents are independently phenyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazolinyl, piperazinyl or 1,3,5-triazolinyl, wherein the nonaromatic nitrogen-containing $R^{17}$ rings are optionally mono-substituted on nitrogen with $(C_1\text{-}C_6)$alkyl, benzyl, benzoyl or $(C_1\text{-}C_6)$alkoxycarbonyl and

wherein the $R^{17}$ rings are optionally mono-substituted on carbon with halo, $(C_1\text{-}C_4)$alkyl, $(C_1\text{-}C_4)$alkoxy, hydroxy, amino, or mono-N- or di-N,N$(C_1\text{-}C_5)$alkylamino provided that no quaternized nitrogen is included and there are no nitrogen-oxygen, nitrogen-nitrogen or nitrogen-halo bonds;

$R^{10}$ is piperazin-1-yl, 4-$(C_1\text{-}C_4)$alkylpiperazin-1-yl, 4-formylpiperazin-1-yl, morpholino, thiomorpholino, 1-oxothiomorpholino, 1,1-dioxo-thiomorpholino, thiazolidin-3-yl, 1-oxo-thiazolidin-3-yl, 1,1-dioxo-thiazolidin-3-yl, 2-$(C_1\text{-}C_6)$alkoxycarbonylpyrrolidin-1-yl, oxazolidin-3-yl or 2(R)-hydroxymethylpyrrolidin-1-yl; or

$R^{10}$ is 3- and/or 4-mono-or di-substituted oxazetidin-2-yl, 2-, 4-, and/or 5- mono- or di-substituted oxazolidin-3-yl, 2-, 4-, and/or 5- mono- or di- substituted thiazolidin-3-yl, 2-, 4-, and/or 5- mono- or di- substituted 1-oxothiazolidin-3-yl, 2-, 4-, and/or 5- mono- or di-substituted 1,1-dioxothiazolidin-3-yl, 3- and/or 4-, mono- or di-substituted pyrrolidin-1-yl, 3-, 4-and/or 5-, mono-, di- or tri-substituted piperidin-1-yl, 3-, 4-, and/or 5- mono-, di-, or tri-substituted piperazin-1-yl, 3-substituted azetidin-1-yl, 4- and/or 5-, mono- or di-substituted 1,2-oxazinan-2-yl, 3-and/or 4-mono- or di-substituted pyrazolidin-1-yl, 4- and/or 5-, mono- or di-substituted isoxazolidin-2-yl, 4- and/or 5-, mono- and/or di-substituted isothiazolidin-2-yl wherein said $R^{10}$ substituents are independently H, halo, $(C_1\text{-}C_5)$-alkyl, hydroxy, amino, mono-N- or di-N,N-$(C_1\text{-}C_5)$alkylamino, formyl, oxo, hydroxyimino, $(C_1\text{-}C_5)$alkoxy, carboxy, carbamoyl, mono-N-or di-N,N-$(C_1\text{-}C_4)$alkylcarbamoyl, $(C_1\text{-}C_4)$alkoxyimino, $(C_1\text{-}C_4)$alkoxymethoxy, $(C_1\text{-}C_6)$alkoxycarbonyl, carboxy$(C_1\text{-}C_5)$alkyl or hydroxy$(C_1\text{-}C_5)$alkyl;

$R^{12}$ is H, methyl, ethyl, n-propyl, hydroxy$(C_1\text{-}C_3)$alkyl, $(C_1\text{-}C_3)$alkoxy$(C_1\text{-}C_3)$alkyl, phenyl$(C_1\text{-}C_4)$alkyl, phenylhydroxy$(C_1\text{-}C_4)$alkyl, (phenyl)($(C_1\text{-}C_4)$-alkoxy)$(C_1\text{-}C_4)$alkyl, thien-2-or -3-yl$(C_1\text{-}C_4)$alkyl or fur-2- or -3-yl$(C_1\text{-}C_4)$ alkyl wherein said $R^{12}$ rings are mono-, di- or tri-substituted independently on carbon with H, halo, $(C_1\text{-}C_4)$ alkyl, $(C_1\text{-}C_4)$alkoxy, trifluoromethyl, hydroxy, amino, cyano or 4,5-dihydro-1 H-imidazol-2-yl; or

$R^{12}$ is pyrid-2-, -3- or -4-yl$(C_1\text{-}C_4)$alkyl, thiazol-2-, -4- or -5-yl$(C_1\text{-}C_4)$alkyl, imidazol-2-,-4- or -5-yl$(C_1\text{-}C_4)$alkyl, pyrrol-2- or -3-yl$(C_1\text{-}C_4)$alkyl, oxazol-2-, -4- or -5-yl$(C_1\text{-}C_4)$alkyl, pyrazol-3-, -4- or -5-yl$(C_1\text{-}C_4)$alkyl, isoxazol-3-, -4- or -5-yl$(C_1\text{-}C_4)$alkyl, isothiazol-3-, -4- or -5-yl$(C_1\text{-}C_4)$alkyl, pyridazin-3- or -4-yl$(C_1\text{-}C_4)$alkyl, pyrimidin-2-, -4-, -5- or -6-yl$(C_1\text{-}C_4)$alkyl, pyrazin-2-or -3-yl$(C_1\text{-}C_4)$alkyl, 1,3,5-triazin-2-yl$(C_1\text{-}C_4)$alkyl or indol-2-$(C_1\text{-}C_4)$ alkyl, wherein said preceding $R^{12}$ heterocycles are optionally mono- or di-substituted independently with halo, trifluoromethyl, $(C_1\text{-}C_4)$alkyl, $(C_1\text{-}C_4)$alkoxy, amino, hydroxy or cyano and said substituents are bonded to carbon; or

$R^{12}$ is $R^{11}$-carbonyloxymethyl, wherein said $R^{11}$ is phenyl, thiazolyl, imidazolyl, 1H-indolyl, furyl, pyrrolyl, oxazolyl, pyrazolyl, isoxazolyl, isothiazolyl, pyridyl, pyridazinyl, pyrimidinyl, pyrazinyl or 1,3,5-triazinyl and wherein said preceding $R^{11}$ rings are optionally mono- or di-substituted independently with halo, amino, hydroxy, $(C_1\text{-}C_4)$alkyl, $(C_1\text{-}C_4)$alkoxy or trifluoromethyl and said mono- or di-substituents are bonded to carbon;

$R^{13}$ is H, methyl, ethyl, n-propyl, hydroxymethyl, or hydroxyethyl;

$R^{14}$ is $C(O)R^{15}$ carboxy, $(C_1\text{-}C_8)$alkoxycarbonyl, benzyloxycarbonyl, or $CONR^{18}R^{19}$;

$R^{15}$ is morpholino, thiomorpholino, 1-oxothiomorpholino, 1,1-dioxothiomorpholino, thiazolidin-3-yl, 1-oxothiazolidin-3-yl, 1,1-dioxothiazolidin-3-yl, pyrrolidin-1-yl, piperidin-1-yl, piperazin-1-yl, piperazin-4-yl, azetidin-1-yl, 1,2-oxazinan-2-yl, pyrazolidin-1-yl, isoxazolidin-2-yl, isothiazolidin-2-yl, 1,2-oxazetidin-2-yl, oxazolidin-3-yl, 3,4-dihydroisoquinolin-2-yl, 1,3-dihydroisoindol-2-yl, 3,4-dihydro-2H-quinol-1-yl, 2,3-dihydro-benzo[1,4]oxazin-4-yl, 2,3-dihydro-benzo[1,4]-thiazine-4-yl, 3,4-dihydro-2H-quinoxalin-1-yl, 3,4-dihydro-benzo[c][1,2]oxazin-1-yl, 1,4-dihydro-benzo[d][1,2]oxazin-3-yl, 3,4-dihydro-benzo[e][1,2]-oxazin-2-yl, 3H-benzo[d]isoxazol-2-yl, 3H-benzo[c]isoxazol-1-yl or azepan-1-yl,

wherein said $R^{15}$ rings are optionally mono-, di- or tri-substituted independently with halo, $(C_1\text{-}C_5)$alkyl, $(C_1\text{-}C_5)$ alkoxy, hydroxy, amino, mono-N- or di-N,N-$(C_1\text{-}C_5)$alkylamino, formyl, carboxy, carbamoyl, mono-N- or di-N,N-$(C_1\text{-}C_5)$alkylcarbamoyl, $(C_1\text{-}C_6)$alkoxy$(C_1\text{-}C_3)$alkoxy, $(C_1\text{-}C_5)$alkoxycarbonyl, benzyloxycarbonyl, $(C_1\text{-}C_5)$ alkoxycarbonyl$(C_1\text{-}C_5)$alkyl, $(C_1\text{-}C_4)$alkoxycarbonylamino, carboxy$(C_1\text{-}C_5)$alkyl, carbamoyl$(C_1\text{-}C_5)$alkyl, mono-N- or di-N,N-$(C_1\text{-}C_5)$alkylcarbamoyl$(C_1\text{-}C_5)$alkyl, hydroxy$(C_1\text{-}C_5)$alkyl, $(C_1\text{-}C_4)$alkoxy$(C_1\text{-}C_4)$alkyl, amino

$(C_1-C_4)$alkyl, mono-N- or di-N,N-$(C_1-C_4)$alkylamino$(C_1-C_4)$alkyl, oxo, hydroxyimino or $(C_1-C_6)$alkoxyimino and wherein no more than two substituents are selected from oxo, hydroxyimino or $(C_1-C_6)$alkoxyimino and oxo, hydroxyimino or $(C_1-C_6)$alkoxyimino are on nonaromatic carbon; and

wherein said $R^{15}$ rings are optionally additionally mono- or di-substituted independently with $(C_1-C_5)$alkyl or halo;

wherein $R^{18}$ is H, $(C_1-C_6)$alkyl, cyclo$(C_3-C_6)$alkyl, cyclo$(C_3-C_6)$alkyl$(C_1-C_5)$alkyl, hydroxy or $(C_1-C_8)$alkoxy; and wherein $R^{19}$ is H, cyclo$(C_3-C_8)$alkyl, cyclo$(C_3-C_8)$alkyl$(C_1-C_5)$alkyl, cyclo$(C_4-C_7)$alkenyl, cyclo$(C_3-C_7)$alkyl $(C_1-C_5)$alkoxy, cyclo$(C_3-C_7)$alkoxy, hydroxy, methylene-perfluorinated$(C_1-C_8)$alkyl, phenyl, or a heterocycle wherein said heterocycle is pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl, 1,3,5-triazinyl, benzothiazolyl, benzoxazolyl, benzimidazolyl, thiochromanyl or tetrahydrobenzothiazolyl wherein said heterocycle rings are carbon-nitrogen linked; or $R^{19}$ is $(C_1-C_6)$alkyl or $(C_1-C_8)$alkoxy wherein said $(C_1-C_8)$alkoxy is optionally monosubstituted with cyclo$(C_4-C_7)$alken-1-yl, phenyl, thienyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, thiomorpholinyl, 1-oxo-thiomorpholinyl, 1,1-dioxothiomorpholinyl, pyridazinyl, pyrimidinyl, pyrazolinyl, piperazinyl, 1,3,5-triazinyl or indolyl and wherein said $(C_1-C_6)$alkyl or $(C_1-C_8)$alkoxy are optionally additionally independently mono- or di-substituted with halo, hydroxy, $(C_1-C_5)$alkoxy, amino, mono-N- or Di-N,N-$(C_1-C_5)$alkylamino, cyano, carboxy, or $(C_1-C_4)$alkoxycarbonyl, and wherein the $R^{19}$ rings are optionally mono- or di-substituted independently on carbon with halo, $(C_1-C_4)$alkyl, $(C_1-C_4)$alkoxy, hydroxy, hydroxy$(C_1-C_4)$alkyl, amino$(C_1-C_4)$alkyl, mono-N- or di-N,N-$(C_1-C_4)$ alkylamino, cyano, carboxy, $(C_1-C_5)$alkoxycarbonyl, carbamoyl, formyl or trifluoromethyl and said $R^{19}$ rings may optionally be additionally mono- or di-substituted independently with $(C_1-C_5)$alkyl or halo, with the proviso that no quaternized nitrogen on any $R^{19}$ heterocycle is included, with the further provisos that when $R^{12}$ is benzyl and $R^{13}$ is methyl, $R^{15}$ is not 4-hydroxy-piperidin-1-yl, and when $R^{12}$ is benzyl and $R^{13}$ is methyl, $R^{15}$ is not $C(O)N(CH_3)_2$.